(19) Europäisches Patentamt / European Patent Office / Office européen des brevets

(11) EP 2 403 950 B1

(12) # FASCICULE DE BREVET EUROPEEN

(45) Date de publication et mention de la délivrance du brevet:
**01.03.2023 Bulletin 2023/09**

(21) Numéro de dépôt: **10709872.5**

(22) Date de dépôt: **02.03.2010**

(51) Classification Internationale des Brevets (IPC):
***C12N 15/82*** *(2006.01)* ***C12N 15/29*** *(2006.01)*
***A01H 5/00*** *(2006.01)* ***C12N 5/04*** *(2006.01)*

(52) Classification Coopérative des Brevets (CPC):
**C12N 15/8289; A01H 5/12; A01H 6/20; C07K 14/415**

(86) Numéro de dépôt international:
**PCT/FR2010/000182**

(87) Numéro de publication internationale:
**WO 2010/100346 (10.09.2010 Gazette 2010/36)**

(54) **PLANTES DU GENRE DIPLOTAXIS PORTEUSES D'UNE STERILITE MALE CYTOPLASMIQUE**

DIPLOTAXUS PFLANZENGATTUNG DIE EINE MÄNNLICHE CYTOPLASMATISCHE STERILITÄT TRAGEN

DIPLOTAXUS PLANT GENUS CARRYING CYTOPLASMATIC MALE STERILITY

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **02.03.2009 FR 0900930**
**09.04.2009 FR 0901763**

(43) Date de publication de la demande:
**11.01.2012 Bulletin 2012/02**

(73) Titulaire: **HM.CLAUSE**
**26800 Portes-les-Valence (FR)**

(72) Inventeurs:
- **HOSEMANS, Danièle**
  **F-49100 Angers (FR)**
- **LEVIEIL, Rémi**
  **F-49124 Saint Barthélemy d'Anjou (FR)**

(74) Mandataire: **Ernest Gutmann - Yves Plasseraud S.A.S.**
**c/o Plasseraud IP**
**66, rue de la Chaussée d'Antin**
**75440 Paris Cedex 09 (FR)**

(56) Documents cités:
**EP-A- 2 111 748 WO-A-2008/084329**
**FR-A- 2 667 078**

- **BANG SANG WOO ET AL: "Production of intergeneric hybrids between the C3-C4 intermediate species Diplotaxis tenuifolia (L.) DC. and Raphanus sativus L." BREEDING SCIENCE, vol. 53, no. 3, septembre 2003 (2003-09), pages 231-236, XP002552640 ISSN: 1344-7610 cité dans la demande**
- **PELLAN-DELOURME R ET AL: "IDENTIFICATION OF MAINTAINER GENES IN BRASSICA-NAPUS L. FOR THE MALE-STERILITY-INDUCING CYTOPLASM OF DIPLOTAXIS-MURALIS L" PLANT BREEDING, PAUL PAREY SCIENTIFIC PUBL., BERLIN, DE, vol. 99, no. 2, 1 janvier 1987 (1987-01-01), pages 89-97, XP002503160 ISSN: 0179-9541**
- **FLANNERY M L ET AL: "Plastid genome characterisation in Brassica and Brassicaceae using a new set of nine SSRs" THEORETICAL AND APPLIED GENETICS ; INTERNATIONAL JOURNAL OF PLANT BREEDING RESEARCH, SPRINGER, BERLIN, DE, vol. 113, no. 7, 15 août 2006 (2006-08-15) , pages 1221-1231, XP019440352 ISSN: 1432-2242**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- **HINATA K ET AL: "Studies on a male sterile strain having the Brassica campestris nucleus and the Diplotaxis muralis cytoplasm. I. On the breeding procedure and some characteristics of the male sterile strain", IKUSHUGAKU ZASSHI - JAPANESE JOURNAL OF BREEDING, TOKYO, JP, vol. 29, no. 4, 1 January 1979 (1979-01-01), pages 305-311, XP002503159, ISSN: 0536-3683**


Remarques:

Le document complet y compris le(s) table(s) de référence et le(s) listage(s) de séquence sont téléchargeables sur le site internet de l'OEB

## Description

La présente invention concerne des plantes de l'espèce *Diplotaxis tenuifolia,* porteuses d'une stérilité mâle cytoplasmique, les graines et les cellules issues de ou donnant naissance à ces plantes.

Les plantes de l'espèce *Diplotaxis tenuifolia* sont connues sous l'appellation générique « roquette ». Cependant, le terme roquette représente en réalité deux genres différents, Eruca et Diplotaxis, qui n'ont de point commun que celui d'appartenir à la même famille des Brassicacées (anciennement nommées Crucifères). La roquette « cultivée » est *Eruca sativa* L. et la roquette « sauvage », *Diplotaxis tenuifolia* L.

La roquette est consommée depuis très longtemps en Europe mais l'espèce dite sauvage n'était pas cultivée mais simplement récoltée dans la nature, d'où les termes « sauvage » et « cultivée » provenant des techniques de production anciennes.

La présente invention concerne plus particulièrement la roquette dite « sauvage » *Diplotaxis tenuifolia.* Il s'agit d'un légume feuille consommé en tant que salade, parfois en mélange comme dans le traditionnel Mesclun, qui apporte une note plus épicée et moins amère que la roquette cultivée.

Le développement récent de la culture industrielle de la mâche, principalement dans la région nantaise, grâce à l'apparition de la quatrième gamme a entraîné le développement de la production de salades « jeunes pousses » puis celui de la roquette. La culture de la roquette dite « sauvage » s'est donc fortement développée ces dernières années en Europe où elle est préférée à la roquette dite « cultivée », et tout particulièrement en Allemagne et en Italie où son développement est très rapide.

Du fait de l'évolution de la production de la roquette, et notamment de l'espèce *Diplotaxis tenuifolia,* l'amélioration de ces plantes est devenue un objectif important des producteurs et des semenciers.

Les plantes du genre Diplotaxis, et notamment de l'espèce *Diplotaxis tenuifolia,* étant préférentiellement allogames, la meilleure solution pour aboutir à des améliorations substantielles au bénéfice des producteurs consiste à créer des variétés dites « hybride F1 ». Les plantes hybrides présentent en effet de meilleurs caractères d'homogénéité aux champs, permettant notamment d'obtenir une récolte uniforme, elles sont aussi plus vigoureuses que les variétés population (ou OP pour « Open pollinated »). De plus, la production d'hybrides permet l'amélioration variétale, des améliorations qualitatives (forme de la feuille, port de la plante, couleur de la feuille, goût...) et des gains de rendement grâce à une meilleure productivité. Elle permet également d'introduire et de cumuler des résistances à certains pathogènes ou prédateurs qui affectent la culture, ainsi que des tolérances à des stress abiotiques.

Cependant, de telles plantes dites « F1 » n'existent pas chez *Oiplotaxis tenuifolia* et ne sont pas mentionnées dans la littérature.

En effet, la production de plantes hybrides F1 n'est économiquement envisageable qu'avec la possibilité de produire des semences commerciales à un coût raisonnable. Ainsi, la pollinisation manuelle (un opérateur prélève le pollen du parent mâle et le dépose sur les organes reproducteurs du parent femelle, tout en s'assurant de la non autopollinisation de la femelle) est d'emblée exclue, ainsi que l'émasculation des fleurs (nécessaire pour assurer l'absence d'autopollinisation).

Concernant la pollinisation entomophile, celle ci n'étant pas dirigée, il faut donc pouvoir disposer d'un système biologique permettant de forcer la pollinisation croisée entre lignées parentales. Par ailleurs, elle suppose également l'émasculation manuelle préalable des fleurs, incompatible avec une production industrielle.

Les présents inventeurs ont donc cherché à apporter des améliorations à la culture de la roquette, grâce à l'obtention de plantes du genre Diplotaxis porteuses d'une stérilité mâle cytoplasmique, et de plantes hybrides F1.

Il existe deux catégories de systèmes naturels utilisables chez les Brassicaceae pour obtenir des plantes hybrides, l'auto-incompatibilité et la stérilité mâle.

Le premier (auto-incompatibilité) est bien connu chez les Brassicacées ; il comporte cependant certaines limites d'utilisation ; sur les Brassicas, l'auto-incompatibilité a été longtemps utilisée ; cependant ce phénomène n'est pas fiable et les lots de semences produits sont souvent des mélanges de graines F1 et de semences issues d'autofécondations.

Le deuxième est basé sur l'absence de pollen viable (stérilité mâle) sur l'une des lignées parentales, ce qui permet l'obtention de lots de semences hybrides à 100%.

Cette stérilité peut être de deux types, génique ou cytoplasmique. La stérilité génique, récessive ou dominante, est parfois utilisée, mais elle est d'une utilisation technique difficile. De plus, aucune stérilité mâle génique n'est connue chez Diplotaxis.

Les présents inventeurs ont donc eu l'idée d'utiliser une stérilité mâle cytoplasmique pour produire des hybrides F1. Cette technique a été utilisée pour produire des hybrides F1, notamment chez le Radis, la Carotte, le Céleri, la Betterave, l'Oignon, le Tournesol, le Fenouil, etc....

Mais pour le genre Diplotaxis, et notamment pour *Diplotaxis tenuifolia,* aucune stérilité mâle cytoplasmique n'a été trouvée au sein des cultivars existants ou dans les populations sauvages, contrairement aux plantes citées plus haut.

Une autre possibilité est d'importer dans le genre Diplotaxis des stérilités mâles cytoplasmiques venant d'espèces « apparentées ».

Cependant, d'après la littérature, il n'existe pas d'espèces du genre Diplotaxis qui soient porteuses d'une stérilité mâle cytoplasmique bien caractérisée qui puisse convenir pour l'importation dans d'autres espèces du genre Diplotaxis.

Les inventeurs ont donc utilisé la stérilité cytoplasmique provenant de plantes d'un genre « apparenté », et non d'une espèce apparentée, en l'occurrence provenant de plantes du genre Raphanus (espèce *Raphanus sativus*), par croisement en suivant des protocoles originaux.

Cette approche est hautement inventive car elle implique d'une part des croisements intergénériques (i.e. entre plantes de genres différents et pas uniquement d'espèces différentes), et il est bien connu de l'homme du métier que de tels croisements sont rarement viables, notamment quand le nombre de chromosomes est différent, ce qui est le cas dans la présente invention, *Raphanus sativus* ayant 2n=18 chromosomes et *Diplotaxis tenuifolia* ayant 2n=22 chromosomes.

De plus, de tels croisements, quand ils sont viables, sont encore plus rarement fertiles. Les inventeurs ont en effet mis en évidence le fait qu'un croisement intergénérique entre des plantes de l'espèce *Brassica rapa,* porteuses d'une stérilité mâle cytoplasmique et des plantes de l'espèce *Diplotaxis tenuifolia* ne permettait pas l'obtention d'une descendance et qu'il en était de même pour un croisement intergénérique entre des plantes *Brassica oleracea* porteuses d'une stérilité mâle cytoplasmique et des plantes *Diplotaxis tenuifolia.*

D'autre part, cette approche implique la cohabitation, dans une même cellule, de génomes chloroplastique et nucléaire provenant de genres différents. En effet, le génome chloroplastique provient du genre dit « apparenté » qui apporte la stérilité mâle cytoplasmique en tant que parent femelle, à savoir le genre Raphanus (*Raphanus sativus*), alors que le génome nucléaire est du genre Diplotaxis. Or chez les crucifères (Brassicacées), le brevet EPO 549 726 enseigne que l'association dans la même cellule de génomes nucléaire et chloroplastique de genres différents entraîne un phénomène de chlorose. Un tel phénomène de chlorose est tout à fait incompatible avec un objectif de commercialisation pour la consommation.

Dans une publication de 2003, Bang S.W. et al décrivent le croisement entre une plante de l'espèce *Diplotaxis tenuifolia* (parent femelle) et une plante de l'espèce *R. sativus* (parent mâle), par sauvetage d'embryons et induction d'amphidiploïdie, suivis de différents croisements avec des plantes D. *tenuifolia* ou *R. sativus.* Il n'est fait aucune mention dans cet article de stérilité mâle cytoplasmique.

Par ailleurs, il est également à noter que les auteurs de l'article indiquent qu'aucun embryon n'a pu être obtenu dans le croisement intergénérique où *R. sativus* est le parent femelle et *Diplotaxis tenuifolia* le parent mâle, ils considèrent donc ce croisement comme étant un croisement incompatible.

La présente invention concerne des plantes mâles stériles, de l'espèce *Diplotaxis tenuifolia,* par le biais d'une stérilité mâle cytoplasmique, leurs différents constituants notamment cellules, les graines issues ou provenant de ces plantes.

Dans le cadre de la présente invention, les termes qui suivent ont plus particulièrement la signification suivante :

**Croisement en retour,** ou **rétro croisement,** ou **backcross,** désigne le croisement d'un hybride avec l'un des deux (2) types parentaux qui ont servi à le former.

**Pourcentage d'identité** de deux séquences : le degré ou pourcentage d'identité entre deux séquences (protéiques ou nucléiques) est déterminé notamment en alignant les deux séquences afin de maximiser les points de concordance tout en minimisant les espaces (« gap ») ; il est obtenu en divisant le nombre d'acides aminés ou d'acides nucléiques communs par la longueur de la plus longue des deux séquences.

La présente invention est dirigée vers une plante de l'espèce *Diplotaxis tenuifolia,* caractérisée en ce qu'elle est porteuse d'une stérilité mâle cytoplasmique, en ce que son génome mitochondrial comprend la séquence d'ADN correspondant aux nucléotides 1673 à 2089 de SEQ ID N°1, ou une séquence ayant au moins 70% d'identité avec cette séquence d'ADN et en ce qu'elle ne présente aucun symptôme de chlorose en conditions normales de culture, notamment pas de décoloration des feuilles due à un manque de chlorophylle.

Diplotaxis est un genre de plantes diploïdes à fleurs, de la famille des Brassicacées, originaire du bassin Méditerranéen.

Par stérilité mâle pour une plante allogame, on entend que ladite plante ne peut pas être parent mâle lors d'un croisement, mais éventuellement parent femelle. Bien qu'il soit parfois fait référence à une plante « mâle » ou « femelle », il est à noter que c'est par abus de langage; dans le cas d'une stérilité mâle, il s'agit en fait d'une fleur hermaphrodite qui a des organes mâles déficients (ou inopérants) et donc qui ne peut être utilisée que comme fleur femelle.

Une stérilité mâle selon la présente invention se caractérise de préférence par l'absence de pollen viable pouvant féconder des organes femelles (organes mâles déficients). Une plante mâle stérile ne peut donc pas participer à un croisement en tant que parent mâle. Une plante mâle stérile selon la présente invention est de préférence parfaitement femelle fertile, c'est-à-dire qu'elle peut participer à un croisement, en tant que parent femelle, avec le même taux de fécondation qu'une plante qui serait mâle fertile.

Une stérilité mâle cytoplasmique est une stérilité mâle portée par le cytoplasme de la plante ou cellule, et plus particulièrement codée par son génome mitochondrial. Le cytoplasme (et donc le génome mitochondrial) étant hérité du parent femelle, toute plante issue du croisement entre une plante selon l'invention comme parent femelle (c'est-à-dire porteuse d'une stérilité mâle cytoplasmique), et d'un parent mâle quel qu'il soit, sera également porteuse de la stérilité mâle cytoplasmique (ou CMS pour « Cytoplasmic Male Sterility ») du parent femelle. Comme une plante selon l'invention ne peut pas être parent mâle dans un croisement, du fait de sa stérilité mâle, toute plante issue par croisement d'une plante selon l'invention, est également porteuse d'une stérilité mâle cytoplasmique.

Il s'agit donc d'un caractère dont nécessairement toute la descendance d'une plante ou graine de l'invention héritera.

La stérilité mâle cytoplasmique selon l'invention est une stérilité stable et fiable, c'est-à-dire que l'ensemble de la descendance d'une plante selon l'invention est mâle stérile.

L'invention concerne une plante de l'espèce *Diplotaxis tenuifolia,* qui est l'espèce la plus répandue du genre Diplotaxis. Il s'agit d'une salade à la saveur piquante et légèrement amère, communément appelée « roquette sauvage ». Une plante de l'espèce *Diplotaxis tenuifolia* est diploïde, avec 2n=22.

Une plante selon l'invention présente un phénotype parfaitement normal, c'est-à-dire un phénotype essentiellement identique à celui d'une plante de la même espèce mâle fertile, la seule différence étant le caractère mâle stérile ou fertile. Pour le sélectionneur, il ne doit pas y avoir de différence phénotypique décelable, statistiquement significative, notamment au niveau de la forme, de la taille, de la couleur ou de la saveur des feuilles. Il ne doit pas y avoir non plus de différence au niveau de la ploïdie.

En particulier, une plante selon la présente invention ne présente aucun symptôme de chlorose en conditions normales de culture, notamment lorsque la température est inférieure à 10°C ; par exemple, quand la température est comprise entre 5 et 10°C. Par chlorose, on entend une décoloration plus ou moins prononcée des feuilles due à un manque de chlorophylle.

En vue d'une commercialisation pour la consommation, il est donc important qu'une plante selon l'invention ne soit pas atteinte de chlorose à quelque stade du développement de la plante que ce soit, dans des conditions normales de culture, spécialement lors de l'abaissement des températures.

Par conditions normales de culture, on entend des conditions de culture qui n'entraînent aucun symptôme de chlorose chez une plante de l'espèce *Diplotaxis tenuifolia* ordinaire (mâle et femelle fertile).

Selon une mise en oeuvre préférée de l'invention, la plante selon l'invention, porteuse de la stérilité mâle cytoplasmique, est homozygote pour l'ensemble de son génome (nucléaire), ou au moins à plus de 90%, voire plus de 95% ou plus de 99%.

Selon une autre mise en oeuvre préférée de l'invention, la plante dont il est question est une plante hybride dite F1. Elle provient de préférence du croisement de deux plantes de la même espèce *Oiplotaxis tenuifolia,* qui proviennent de deux lignées phénotypiquement différentes.

Quoi qu'il en soit, le parent femelle d'une plante hybride F1 selon l'invention est une plante de l'espèce *Diplotaxis tenuifolia,* porteuse de la stérilité mâle cytoplasmique.

La stérilité mâle cytoplasmique est connue à l'état naturel chez l'espèce *Raphanus sativus* (radis) et cette stérilité mâle a déjà été exploitée sous le nom de stérilité mâle cytoplasmique Ogura. Elle a notamment été transmise à différentes espèces du genre Brassica, comme montré dans le brevet EPO 549 726. Elle se caractérise alors par la séquence d'ADN comprise entre les nucléotides 928 et 2273 de la figure 1 du brevet EPO 549 726 ou une séquence ayant au moins 50% d'identité avec ladite séquence et conférant une stérilité mâle cytoplasmique quand elle est présente dans le génome mitochondrial d'une plante.

La stérilité mâle cytoplasmique selon la présente invention est une stérilité issue de la stérilité mâle cytoplasmique de *Raphanus sativus,* ce qui se traduit par la présence dans le génome mitochondrial d'une plante de l'invention, de séquences originaires de R. *sativus,* plus particulièrement du génome mitochondrial de *R. sativus.* Lesdites séquences provenant du génome mitochondrial de *Raphanus sativus* représentent de préférence au moins 50% du génome mitochondrial selon l'invention, ou sont d'une longueur d'au moins 50, voire d'au moins 100, 200, 500 ou 1000 paires de bases consécutives.

Dans une mise en oeuvre préférée, le génome mitochondrial d'une plante de l'invention comprend au moins une partie de la séquence d'ADN illustrée à la figure 1 (SEQ ID N°1 correspondant à Z18896, qui comprend l'orf138 de R. sativus mâle stérile Ogura) cette partie comprenant au moins 50 paires de bases consécutives de SEQ ID N°1. De préférence, ladite partie de SEQ ID N°1 a une longueur d'au moins 100, de préférence au moins 200, 500, 1000 ou 2000 paires de bases.

Dans une autre mise en oeuvre préférée, le génome mitochondrial d'une plante de l'invention comprend au moins une séquence ayant au moins 70% d'identité avec une partie de la séquence d'ADN illustrée à la figure 1 (SEQ ID N°1), cette partie comprenant au moins 500 paires de bases consécutives de SEQ ID N°1, de préférence au moins 1000 ou 2000 paires de bases. De préférence également, ladite séquence présente au moins 80% d'identité, voire au moins 90% ou 95% d'identité ou même au moins 99% d'identité avec ladite partie de SEQ ID N°1.

De manière particulièrement préférée, la partie de SEQ ID N°1 présente dans le génome d'une plante selon l'invention confère la stérilité mâle cytoplasmique.

Quoi qu'il en soit, le génome mitochondrial d'une plante de l'invention comprend au moins la partie correspondant aux nucléotides 1673 à 2089 de SEQ ID N°1, qui comprennent l'orf138, ou une séquence ayant au moins 70% d'identité avec cette partie. Cette séquence est en effet caractéristique de la stérilité mâle cytoplasmique dite Ogura. Alternativement, ladite partie est un fragment de 512 paires de bases correspondant aux nucléotides 1579 à 2090 de SEQ ID N°1 comprenant l'orf138. De préférence, le génome mitochondrial d'une plante de l'invention comprend un fragment de 401 paires de bases correspondant aux nucléotides 1248 à 1648 de SEQ ID N°1, comprenant tout ou partie du gène T-ARNt (Transfer-ARNt formylmetionine).

De ce fait, une plante selon l'invention se caractérise de préférence par un fragment amplifié de la taille suivante, quand les amorces correspondantes sont utilisées pour amplifier par PCR le génome mitochondrial de la plante :

- 512 paires de bases quand les amorces oligo 37 et 38 (respectivement SEQ ID N°22 et 23) sont utilisées et/ou
- 401 paires de bases quand les amorces TRNAFM-610U et TRNAFM-987L (respectivement SEQ ID N°24 et 25) sont utilisées.

De préférence, la séquence du fragment amplifié de 512 paires de bases est SEQ ID N°20 et cette séquence est présente dans le génome d'une plante selon l'invention.

De préférence, la séquence du fragment amplifié de 401 paires de bases est SEQ ID N°21 et cette séquence est présente dans le génome d'une plante selon l'invention.

Par ailleurs, dans un mode de réalisation préféré de la présente invention, une plante telle que décrite porteuse d'une stérilité mâle cytoplasmique se distingue également au niveau du génome chloroplastique, d'une autre plante qui ne serait pas porteuse de ladite stérilité.

De préférence, une plante de l'invention comporte dans ses chloroplastes, au niveau de l'ADN chloroplastique, des séquences qui ne sont pas du genre Diplotaxis. Par exemple, au moins 200 paires de bases du génome chloroplastique ne sont pas du genre Diplotaxis, de préférence au moins 500 voire un millier de bases. Dans une situation préférée, l'intégralité du génome chloroplastique n'est pas celui du genre Diplotaxis.

La partie du génome chloroplastique qui n'est pas du genre Diplotaxis est de préférence de l'espèce *R. sativus.* De manière préférée, il s'agit de tout ou partie du génome chloroplastique de *Raphanus sativus.* Comme précisé plus haut, lesdites séquences s'entendent de séquences d'ADN comprenant au moins 200 ou au moins 500 paires de bases consécutives. De préférence, le génome chloroplastique d'une plante selon l'invention provient de l'espèce *Raphanus sativus,* il ne contient donc pas de séquence caractéristique du génome chloroplastique de *B. rapa* ou *B. oleracea,* ou de tout autre Brassica.

Selon un mode de réalisation plus particulièrement illustré dans la partie expérimentale de cette demande, une plante selon l'invention se caractérise par des cellules qui possèdent l'ADN génomique de *Diplotaxis tenuifolia,* l'ADN mitochondrial de *Raphanus sativus* stérilité Ogura et l'ADN chloroplastique de *Raphanus sativus.*

Des protocoles permettant la détermination de séquences au sein du génome mitochondrial et chloroplastique d'une cellule de plantes sont décrits dans la partie expérimentale (voir exemple 2 pour le génome chloroplastique et le génome mitochondrial). Ils peuvent être mis en oeuvre pour déterminer si une plante donnée, mâle stérile de manière cytoplasmique, comprend la stérilité dite Ogura dans son ADN mitochondrial et comprend de l'ADN chloroplastique de *Raphanus sativus.*

Une plante selon l'invention se caractérise de préférence par un fragment amplifié de la taille suivante, quand les amorces correspondantes sont utilisées pour amplifier par PCR le génome chloroplastique de la plante :

- 193 paires de bases quand les amorces oligo MF2_M13F et MF2_R (respectivement SEQ ID N°4 et 5) sont utilisées et/ou
- 264 paires de bases quand les amorces MF8_M13F et MF8_R (respectivement SEQ ID N°16 et 17) sont utilisées et/ou
- 180 paires de bases quand les amorces MF7_M13F et MF7_R (respectivement SEQ ID N°14 et 15) sont utilisées et/ou
- 319 paires de bases quand les amorces MF9_M13F et MF9_R (respectivement SEQ ID N°18 et 19) sont utilisées et/ou
- 303 paires de bases quand les amorces MF3_M13F et MF3_R (respectivement SEQ ID N°6 et 7) sont utilisées et/ou
- 183 paires de bases quand les amorces MF6_M13F et MF6_R (respectivement SEQ ID N°12 et 13) sont utilisées.

Lesdites amorces s'hybrident en effet avec le génome chloroplastiques des plantes D. tenuifolia, R. sativus CMS et B. oleracea CMS ogura, mais la taille du fragment amplifié varie et caractérise les plantes selon l'invention, dont le cytoplasme est originaire de R. *sativus CMS,* comme il ressort de l'exemple 2.

Pour déterminer si une plante mâle stérile est porteuse d'une stérilité cytoplasmique ou génique, un protocole de test simple consiste à faire des croisements avec une plante fertile et à déterminer la proportion de la descendance qui est également mâle stérile. Dans le cas d'une stérilité mâle cytoplasmique, toute plante issue de ce croisement est également mâle stérile, ainsi que toute plante issue de ce croisement par backcross avec la plante fertile.

Dans le cas d'une stérilité génique récessive, en règle générale, aucune plante issue de ce croisement n'est mâle stérile.

Dans le cas d'une stérilité génique dominante, la moitié des plantes issues de ce croisement sont également mâles stériles. En effet, si la stérilité génique est dominante, le parent femelle ne peut être qu'hétérozygote pour ce gène de stérilité et donc ne transmettre ce trait qu'à la moitié de la descendance. La ségrégation s'effectue donc de façon mendélienne dans la descendance dans le cas d'une stérilité génique, alors qu'il n'y a pas de ségrégation dans le cas d'une stérilité cytoplasmique, l'hérédité étant femelle.

Il est à noter que la distinction entre une plante mâle stérile (cytoplasmique) selon l'invention et une plante mâle fertile, peut être réalisée à l'œil nu ; en effet, les plantes sont phénotypiquement différentes du fait de l'absence de pollen dans le cas des plantes mâles stériles.

Les plantes selon la présente invention sont de préférence obtenues sans avoir recours à la fusion de protoplastes, et sans induction d'un doublement du nombre de chromosomes à un quelconque stade d'obtention, afin de préserver la diploïdie.

La présente invention concerne également des graines de l'espèce *Diplotaxis tenuifolia,* et plus particulièrement des graines porteuses d'une stérilité mâle cytoplasmique et dont le génome mitochondrial comprend la séquence d'ADN correspondant aux nucléotides 1673 à 2089 de SEQ ID N°1, ou une séquence ayant au moins 70% d'identité avec cette séquence d'ADN. L'invention concerne notamment des graines qui, après germination, permettent d'obtenir une plante telle que décrite plus haut, c'est-à-dire porteuse d'une stérilité mâle cytoplasmique et de l'espèce *Diplotaxis tenuifolia.* Elle concerne également des graines issues du croisement d'une plante telle que décrite plus haut, avec une autre plante de l'espèce *Diplotaxis tenuifolia,* qui soit mâle fertile. Comme indiqué plus haut, toute graine issue du croisement d'une plante selon l'invention et d'une autre plante de l'espèce *Diplotaxis tenuifolia* donnera également naissance à une plante de l'espèce *Diplotaxis tenuifolia* porteuse d'une stérilité mâle cytoplasmique.

Il a déjà été défini plus haut ce qu'il faut entendre par « stérilité mâle cytoplasmique », dans le cadre des plantes de l'invention, et les définitions et mises en oeuvres préférées sont les mêmes pour ce qui concerne les graines selon l'invention.

Il est à noter que la stérilité mâle cytoplasmique dont il est fait référence est une stérilité stable et fiable. De manière tout particulièrement préférée, cette stérilité mâle ne s'accompagne pas d'une stérilité femelle. En effet, des graines et plantes préférées dans le cadre de la présente invention sont femelle-fertiles.

Une graine selon l'invention est une graine de l'espèce *Diplotaxis tenuifolia,* et par conséquent diploïde avec 2n=22.

Une graine selon l'invention comprend dans son ADN mitochondrial des séquences provenant de *Raphanus sativus,* comme c'est le cas pour les plantes de l'invention, et plus particulièrement des séquences du génome mitochondrial de *R. sativus CMS.* En effet, la stérilité mâle cytoplasmique est issue de la stérilité mâle cytoplasmique de R. *sativus,* ce qui se traduit par la présence dans le génome mitochondrial d'une graine de l'invention, de séquences originaires de *R. sativus CMS.*

Lesdites séquences provenant du génome mitochondrial de *Raphanus sativus* représentent de préférence au moins 50% du génome mitochondrial d'une graine selon l'invention, de préférence au moins 80%, ou sont d'une longueur d'au moins 50, voire d'au moins 100, 200, 500 ou 1000 paires de bases consécutives.

Le génome mitochondrial d'une graine comprend la séquence d'ADN comprise entre les nucléotides 1673 et 2089 de la figure 1 (SEQ ID N°1) ou une séquence ayant au moins 70% d'identité avec ladite séquence et conférant la stérilité mâle cytoplasmique. De préférence, le génome mitochondrial d'une graine selon l'invention comprend une séquence ayant au moins 80%, voire au moins 90% ou 95% d'identité ou au moins 99% d'identité avec ladite séquence d'ADN.

De manière particulièrement préférée, la partie de SEQ ID N°1 présente dans le génome d'une graine selon l'invention confère la stérilité mâle cytoplasmique.

De préférence, ladite partie correspond aux nucléotides 1673 à 2089 de SEQ ID N°1, qui comprennent l'orf138. Cette séquence est en effet caractéristique de la stérilité mâle cytoplasmique dite Ogura. Alternativement, ladite partie est un fragment de 512 paires de bases correspondant aux nucléotides 1579 à 2090 de SEQ ID N°1 comprenant l'orf138. De préférence, le génome mitochondrial d'une graine de l'invention comprend un fragment de 401 paires de bases correspondant aux nucléotides 1248 à 1648 de SEQ ID N°1, comprenant tout ou partie du gène T-ARNt (Transfer-ARNt formylmetionine).

De ce fait, une graine selon l'invention se caractérise de préférence par un fragment amplifié de la taille suivante, quand les amorces correspondantes sont utilisées pour amplifier par PCR son génome mitochondrial:

- 512 paires de bases quand les amorces oligo 37 et 38 (respectivement SEQ ID N°22 et 23) sont utilisées et/ou
- 401 paires de bases quand les amorces TRNAFM-610U et TRNAFM-987L (respectivement SEQ ID N°24 et 25) sont utilisées.

De préférence, la séquence du fragment amplifié de 512 paires de bases est SEQ ID N°20 et cette séquence est présente dans le génome d'une graine selon l'invention.
De préférence, la séquence du fragment amplifié de 401 paires de bases est SEQ ID N°21 et cette séquence est présente dans le génome d'une graine selon l'invention.
La séquence mitochondriale initialement décrite comme liée à la CMS Ogura chez les plantes du genre Brassica est la séquence mentionnée dans le brevet EP 0 549 726 ; des variants de cette séquence ont depuis été décrits, notamment sous les numéros d'accession Z12626 et Z18896 (SEQ ID N°1).
Par ailleurs, dans un mode de réalisation préféré, une graine telle que décrite porteuse d'une stérilité mâle cytoplasmique se distingue également au niveau du génome chloroplastique, d'une autre graine qui ne serait pas porteuse de ladite stérilité via un croisement intergénérique avec une plante du genre Raphanus.
De préférence, une graine de l'invention comporte dans ses chloroplastes, au niveau de l'ADN chloroplastique, des séquences qui ne sont pas du genre Diplotaxis. Par exemple, au moins 200 paires de bases du génome chloroplastique ne sont pas du genre Diplotaxis, de préférence au moins 500, de préférence encore au moins un millier de bases. Dans une situation préférée, l'intégralité du génome chloroplastique n'est pas celui du genre Diplotaxis.
La partie du génome chloroplastique qui n'est pas du genre Diplotaxis est de préférence du genre Raphanus. De manière particulièrement préférée, il s'agit de séquences provenant du génome chloroplastique de *Raphanus sativus.* Comme précisé plus haut, lesdites séquences s'entendent de séquences d'ADN comprenant au moins 200 paires de bases consécutives, de préférence au moins 500 paires de bases. De préférence, le génome chloroplastique d'une graine selon l'invention provient de l'espèce *Raphanus sativus,* il ne contient donc pas de séquence caractéristique du génome chloroplastique de *B. rapa* ou *B. oleracea,* ou de tout autre Brassica.
Une graine selon l'invention se caractérise de préférence par un fragment amplifié de la taille suivante, quand les amorces correspondantes sont utilisées pour amplifier par PCR son génome chloroplastique :

- 193 paires de bases quand les amorces oligo MF2_M13F et MF2_R (respectivement SEQ ID N°4 et 5) sont utilisées et/ou
- 264 paires de bases quand les amorces MF8_M13F et MF8_R (respectivement SEQ ID N°16 et 17) sont utilisées et/ou
- 180 paires de bases quand les amorces MF7_M13F et MF7_R (respectivement SEQ ID N°14 et 15) sont utilisées et/ou
- 319 paires de bases quand les amorces MF9_M13F et MF9_R (respectivement SEQ ID N°18 et 19) sont utilisées et/ou
- 303 paires de bases quand les amorces MF3_M13F et MF3_R (respectivement SEQ ID N°6 et 7) sont utilisées et/ou
- 183 paires de bases quand les amorces MF6_M13F et MF6_R (respectivement SEQ ID N°12 et 13) sont utilisées.

L'exemple 2 illustre ce point.
La présente invention concerne aussi des cellules de plantes, qui soient porteuses d'une stérilité mâle cytoplasmique, de l'espèce *Diplotaxis tenuifolia* et dont le génome mitochondrial comprend la séquence d'ADN correspondant aux nucléotides 1673 à 2089 de SEQ ID N°1, ou une séquence ayant au moins 70% d'identité avec cette séquence d'ADN.
De préférence, une cellule selon la présente invention est issue d'une plante ou bien d'une graine telles que décrites précédemment. Il peut s'agir d'une cellule à tout stade de développement ou de différenciation. Il peut s'agir de cellules reproductrices, de cellules de cal, du système racinaire, foliaire, de l'apex, du méristème, ou de toute autre partie de la plante.
La cellule en question peut être isolée, en amas ou au sein d'un tissu. Elle peut être en suspension ou sur un milieu solide. Une cellule dans le cadre de l'invention est une cellule de l'espèce *Diplotaxis tenuifolia* et donc diploïde, avec 2n=22.
Comme explicité plus haut en rapport avec les plantes et les graines, une cellule de l'invention comprend dans son génome mitochondrial des séquences provenant de *R. sativus,* plus particulièrement des séquences provenant du génome mitochondrial de *R. sativus* porteur de la stérilité mâle cytoplasmique dite Ogura. Lesdites séquences provenant du génome mitochondrial de *R. sativus* représentent de préférence au moins 50% du génome mitochondrial d'une cellule selon l'invention, de préférence au moins 80%, ou sont d'une longueur d'au moins 50, voire d'au moins 100, 200, 500 ou 1000 paires de bases consécutives.
Le génome mitochondrial d'une cellule comprend la séquence d'ADN comprise entre les nucléotides 1673 et 2089 de la figure 1 (SEQ ID N°1) correspondant à l'orf138 ou une séquence ayant au moins 70% d'identité avec ladite séquence et conférant la stérilité mâle cytoplasmique. De préférence, le génome mitochondrial d'une cellule selon l'invention comprend une séquence ayant au moins 80%, voire au moins 90% ou 95% d'identité ou au moins 99% d'identité avec ladite séquence d'ADN, c'est-à-dire qu'elle possède la stérilité mâle cytoplasmique dite stérilité mâle Ogura telle que mise en oeuvre dans le brevet EPO 549 726 de l'INRA.

De manière particulièrement préférée, la partie de SEQ ID N°1 présente dans le génome d'une plante selon l'invention confère la stérilité mâle cytoplasmique. De préférence, ladite partie correspond aux nucléotides 1673 à 2089 de SEQ ID N°1, qui comprennent l'orf138. Cette séquence est en effet caractéristique de la stérilité mâle cytoplasmique dite stérilité Ogura. Alternativement, ladite partie est un fragment de 512 paires de bases correspondant aux nucléotides 1579 à 2090 de SEQ ID N°1 comprenant l'orf138. De préférence, le génome mitochondrial d'une cellule de l'invention comprend un fragment de 401 paires de bases correspondant aux nucléotides 1248 à 1648 de SEQ ID N°1, comprenant tout ou partie du gène T-ARNt (Transfer-ARNt formylmetionine).

De ce fait, une cellule selon l'invention se caractérise de préférence par un fragment amplifié de la taille suivante, quand les amorces correspondantes sont utilisées pour amplifier par PCR son génome mitochondrial :

- 512 paires de bases quand les amorces oligo 37 et 38 (respectivement SEQ ID N°22 et 23) sont utilisées et/ou
- 401 paires de bases quand les amorces TRNAFM-610U et TRNAFM-987L (respectivement SEQ ID N°24 et 25) sont utilisées.

De préférence, la séquence du fragment amplifié de 512 paires de bases est SEQ ID N°20 et cette séquence est présente dans le génome d'une cellule selon l'invention.

De préférence, la séquence du fragment amplifié de 401 paires de bases est SEQ ID N°21 et cette séquence est présente dans le génome d'une cellule selon l'invention.

Par ailleurs, dans un mode de réalisation préféré, une cellule de plante telle que décrite porteuse d'une stérilité mâle cytoplasmique se distingue également au niveau du génome chloroplastique, d'une autre cellule de l'espèce *Diplotaxis tenuifolia.* Notamment, une cellule de l'invention comporte de préférence dans son ADN chloroplastique, des séquences qui ne sont pas du genre Diplotaxis. Comme déjà explicité en rapport avec les plantes et graines de l'invention, il peut s'agir par exemple, d'au moins 200 paires de bases du génome chloroplastique qui ne sont pas du genre Diplotaxis, de préférence au moins 500, de préférence encore au moins un millier de bases. Dans une situation préférée, l'intégralité du génome chloroplastique n'est pas celui du genre Diplotaxis.

La partie du génome chloroplastique qui n'est pas du genre Diplotaxis est préférentiellement du genre Raphanus. De manière particulièrement préférée, il s'agit de séquences provenant du génome chloroplastique de *Raphanus sativus.* Comme précisé plus haut, lesdites séquences s'entendent de séquences d'ADN comprenant au moins 200 paires de bases consécutives, voire au moins 500.

De préférence, le génome chloroplastique d'une cellule selon l'invention provient de l'espèce *Raphanus sativus,* il ne contient donc pas de séquence caractéristique du génome chloroplastique de B. *rapa* ou B. *oleracea,* ou de tout autre Brassica.

Une cellule selon l'invention se caractérise de préférence par un fragment amplifié de la taille suivante, quand les amorces correspondantes sont utilisées pour amplifier par PCR le génome chloroplastique :

- 193 paires de bases quand les amorces oligo MF2_M13F et MF2_R (respectivement SEQ ID N°4 et 5) sont utilisées et/ou
- 264 paires de bases quand les amorces MF8_M13F et MF8_R (respectivement SEQ ID N°16 et 17) sont utilisées et/ou
- 180 paires de bases quand les amorces M F7 _M 13F et MF7_R (respectivement SEQ ID N°14 et 15) sont utilisées et/ou
- 319 paires de bases quand les amorces MF9_M13F et MF9_R (respectivement SEQ ID N°18 et 19) sont utilisées et/ou
- 303 paires de bases quand les amorces MF3_M13F et MF3_R (respectivement SEQ ID N°6 et 7) sont utilisées et/ou
- 183 paires de bases quand les amorces MF6_M13F et MF6_R (respectivement SEQ ID N°12 et 13) sont utilisées.

La présente description se rapporte également à différents procédés, non revendiqués, permettant d'obtenir des plantes, cellules ou graines telles que décrites dans les parties précédentes.

La description a notamment trait, selon un aspect non revendiqué, à un procédé d'obtention d'une plante ou d'une graine, de préférence de l'espèce *Diplotaxis tenuifolia,* comprenant :

a) le croisement intergénérique d'une plante *Raphanus sativus,* porteuse d'une stérilité mâle cytoplasmique, comme parent femelle, et d'une plante *Diplotaxis tenuifolia* mâle fertile, comme parent mâle ;
b) l'obtention d'une plante issue du croisement précédent par sauvetage d'embryons ;
c) le croisement d'une plante issue de l'étape b), comme parent femelle, avec une plante *Diplotaxis tenuifolia* mâle fertile, comme parent mâle ;

d) l'obtention d'une plante issue du croisement c), éventuellement par sauvetage d'embryons.

Des plantes *Raphanus sativus* porteuses d'une stérilité mâle cytoplasmique, sont disponibles dans le commerce (par exemple : variété Clémentine). Il en est de même des plantes *Oiplotaxis tenuifolia* (mâle fertile), permettant de réaliser le premier croisement intergénérique.

Les inventeurs ont démontré qu'un tel croisement intergénérique, peut conduire à l'obtention d'hybrides viables, et mâles stériles. Aux fins d'une telle obtention, il est toutefois nécessaire, après fécondation, de prélever les siliques puis les ovules et de les mettre en culture *in vitro,* c'est-à-dire de procéder à une étape de sauvetage d'embryons. Par cette technique, il est ainsi possible d'obtenir et de développer une plante correspondant au croisement intergénérique d'une plante *Raphanus sativus,* porteuse d'une stérilité mâle cytoplasmique, comme parent femelle, et d'une plante *Diplotaxis tenuifolia* mâle fertile, comme parent mâle.

Il est toutefois à noter que l'obtention d'une plante issue de ce croisement peut nécessiter de réaliser un nombre important de croisements et de mises en culture d'ovules. Les inventeurs ont toutefois répété plusieurs fois ce croisement intergénérique et ont montré la faisabilité, la viabilité et la fertilité (femelle) d'un tel croisement (voir exemple 1).

Il est à noter à ce propos que les deux espèces ne possèdent pas le même nombre de chromosomes (2n=22 pour *Diplotaxis tenuifolia* et 2n=18 pour *Raphanus sativus*).

Le procédé comprend ensuite un nouveau croisement en retour (backcross) avec une plante de l'espèce D. *tenuifolia* utilisée comme parent mâle, et de nouveau l'obtention d'une plante issue de ce croisement. A ce stade également il peut être nécessaire de procéder à un sauvetage d'embryons dans le cas où aucun ovule ne se développe *in vivo.* Ces étapes de rétro-croisement et obtention d'une plante (étapes c et d du procédé) peuvent être répétées plusieurs fois. Elles sont de préférence mises en oeuvre au moins deux voire au moins trois fois, afin que la plante obtenue soit phénotypiquement de l'espèce *D. tenuifolia,* ou bien qu'il s'agisse d'une graine également de l'espèce *D. tenuifolia,* donc diploïde et que son nombre de chromosomes soit équivalent à celui de *D. tenuifolia,* c'est-à-dire 2n=22.

Après la première ou deuxième mise en oeuvre des étapes c et d, il est généralement inutile de procéder au sauvetage d'embryons pour obtenir une plante viable.

Selon la mise en oeuvre préférée du procédé, les plantes sont obtenues sans qu'aucune étape de fusion de protoplastes ne soit réalisée. De préférence, il n'y a également aucune étape d'induction d'un doublement du nombre de chromosomes. Il n'est toutefois pas exclu qu'un doublement du nombre de chromosomes ait lieu naturellement, mais de préférence, il n'y en a pas.

Les plantes obtenues par ce procédé ou bien les graines qui en sont issues, sont porteuses de la stérilité mâle cytoplasmique.

L'invention concerne également les plantes, graines et cellules, qui sont susceptibles d'être obtenues par la mise en oeuvre du procédé tel que décrit, ainsi que tout matériel biologique qui en est issu.

Une plante, graine ou cellule susceptible d'être obtenue par la mise en oeuvre du procédé tel que décrit, se caractérise notamment au niveau de son génome mitochondrial, porteur de la stérilité mâle cytoplasmique, qui comprend la partie correspondant aux nucléotides 1673 à 2089 de SEQ ID N°1 (ORF138).

Une plante, graine ou cellule susceptible d'être obtenue par la mise en oeuvre du procédé tel que décrit, se caractérise notamment au niveau de son génome mitochondrial, de préférence par un fragment amplifié de la taille suivante, quand les amorces correspondantes sont utilisées pour amplifier par PCR le génome mitochondrial :

- 512 paires de bases quand les amorces oligo 37 et 38 (respectivement SEQ ID N°22 et 23) sont utilisées et/ou
- 401 paires de bases quand les amorces TRNAFM-610U et TRNAFM-987L (respectivement SEQ ID N°24 et 25) sont utilisées.

De préférence, la séquence amplifiée de 512 paires de bases est SEQ ID N°20 et cette séquence est présente dans le génome d'une plante, graine ou cellule susceptible d'être obtenue par la mise en oeuvre du procédé de l'invention.

De même, de préférence la séquence amplifiée de 401 paires de bases est SEQ ID N°21 et cette séquence est présente dans le génome d'une plante, graine ou cellule susceptible d'être obtenue par la mise en oeuvre du procédé de l'invention.

Une plante, graine ou cellule susceptible d'être obtenue par la mise en oeuvre du procédé tel que décrit, se caractérise également au niveau de son génome chloroplastique, de préférence par un fragment amplifié de la taille suivante, quand les amorces correspondantes sont utilisées pour amplifier par PCR le génome chloroplastique :

- 193 paires de bases quand les amorces oligo MF2_M13F et MF2_R (respectivement SEQ ID N°4 et 5) sont utilisées et/ou
- 264 paires de bases quand les amorces MF8_M13F et MF8_R (respectivement SEQ ID N°16 et 17) sont utilisées et/ou

- 180 paires de bases quand les amorces MF7_M13F et MF7_R (respectivement SEQ ID N°14 et 15) sont utilisées et/ou
- 319 paires de bases quand les amorces MF9_M13F et MF9_R (respectivement SEQ ID N°18 et 19) sont utilisées et/ou
- 303 paires de bases quand les amorces MF3_M13F et MF3_R (respectivement SEQ ID N°6 et 7) sont utilisées et/ou
- 183 paires de bases quand les amorces MF6_M13F et MF6_R (respectivement SEQ ID N°12 et 13) sont utilisées.

De préférence, le génome chloroplastique d'une plante, graine ou cellule susceptible d'être obtenue par la mise en oeuvre du procédé tel que décrit, provient de l'espèce *Raphanus sativus,* il ne contient donc pas de séquence caractéristique du génome chloroplastique de *B. rapa* ou *B. oleracea,* ou de tout autre Brassica.

Concernant le sauvetage d'embryons éventuellement utilisé dans le procédé, il s'agit d'une technique bien connue de l'homme du métier et régulièrement pratiquée dans le cas de croisements intergénériques ou plus couramment interspécifiques. Cette technique est plus particulièrement décrite dans la publication Rahman (2004) et sa mise en oeuvre est détaillée dans la section expérimentale de la demande (exemple 1).

Les différents milieux, conditions et temps de culture, pour la mise en oeuvre de cette technique, sont décrits dans la littérature et peuvent être adaptés ou modifiés si nécessaire. Des exemples en sont donnés dans la partie expérimentale.

La présente description concerne également, dans un aspect non revendiqué, l'utilisation d'une plante telle que décrite dans le cadre de la présente demande, comme partenaire femelle dans un croisement avec une autre plante du genre Diplotaxis mâle fertile. Par exemple, une plante de l'espèce *D. tenuifolia,* telle que décrite dans la demande, peut être croisée avec une plante du genre Diplotaxis, qui ne soit pas mâle stérile.

Ainsi, grâce à l'utilisation d'une plante *D. tenuifolia* porteuse d'une stérilité mâle cytoplasmique, il est possible par exemple, par des programmes de sélection, d'obtenir pour chaque sous-espèce ou variété de *D. tenuifolia* connue, une sous-espèce ou variété équivalente qui soit mâle stérile. Il est également possible d'obtenir des plantes du genre Diplotaxis mais d'autres espèces que *D. tenuifolia,* en réalisant un premier croisement interspécifique, suivi de différents croisements en retour. La stérilité mâle cytoplasmique d'une plante selon l'invention peut donc être utilisée comme vecteur de la stérilité mâle cytoplasmique chez une autre plante de Diplotaxis, ou chez une plante *Diplotaxis tenuifolia* d'une autre sous-espèce ou variété.

La description concerne également, selon un aspect non revendiqué, l'utilisation d'une plante de l'espèce *Raphanus sativus,* porteuse d'une stérilité mâle cytoplasmique, de préférence la stérilité mâle dite Ogura, comme vecteur de la stérilité mâle cytoplasmique chez une plante de Diplotaxis, de préférence chez une plante *Diplotaxis tenuifolia.* Comme démontré dans la partie expérimentale qui suit, de manière surprenante, les inventeurs ont en effet découvert qu'il était possible de transférer la stérilité mâle cytoplasmique connue de *Raphanus sativus* chez des plantes de l'espèce *Diplotaxis tenuifolia,* grâce à un croisement intergénérique uniquement, et sans recours à la fusion de protoplastes.

Ils ont par ailleurs démontré que les plantes obtenues n'étaient pas atteintes de chlorose, et ce contrairement à l'enseignement du brevet EP 0 549 726 qui correspond au préjugé répandu chez les personnes travaillant dans le domaine de l'invention.

Les inventeurs ont donc obtenu pour la première fois une plante de la famille des Brassicacées mais d'un genre autre que Brassica, qui soit porteuse d'une stérilité mâle cytoplasmique issue de *Raphanus sativus,* et dont le génome chloroplastique soit en partie celui de *Raphanus sativus,* c'est-à-dire qu'il comprend au moins 200 paires de bases consécutives de *Raphanus sativus,* de préférence au moins 500, et ce sans être atteinte de chlorose, notamment à des températures inférieures à 10°C.

**Légende des figures.**

Figure 1 : Cette figure illustre la séquence SEQ ID N°1 correspondant au numéro d'accession Z18896, qui comprend la séquence de l'orf138 du génome mitochondrial de Raphanus sativus CMS. Les séquences correspondant aux amorces oligo 37 et 38 (respectivement SEQ ID N°22 et 23) sont soulignées par un double trait, les séquence correspondant aux amorces TRNAFM-610U et TRNAFM-987L (respectivement SEQ ID N°24 et 25) sont soulignées par un simple trait.

Figure 2 : Cette figure illustre le résultat de la migration électrophorétique sur gel d'agarose de différents échantillons d'ADN, après amplification PCR avec les amorces oligo 37 et oligo 38 (orf138, gel supérieur), avec les amorces TRNAFM-610U et TRNAFM-987L (T-ARNt, gel du milieu) et avec les amorces COX1-244U et COX1-805L (cox1, gel du bas). Le trois premiers puits (M1, M2 et M6) correspondent à des échantillons d'ADN provenant de trois lignées de *R. sativus* CMS, les 4$^{ème}$, 6$^{ème}$ et 8$^{ème}$ puits (M7, M9 et M11 respectivement) correspondent à des échantillons d'ADN provenant de lignées de *D. tenuifolia* fertiles; les 5ème, 7ème et 9ème puits (M8, M10 et M12 respectivement) correspondent à des échantillons d'ADN provenant de différentes plantes *D. tenuifolia* mâles stériles selon l'invention.
F= fertile ; S= stérile.

**PARTIE EXPERIMENTALE**

**Exemple 1 : obtention de plantes *D. tenuifolia* mâles stériles (cytoplasmique)**

*Résumé des travaux*

Les inventeurs ont croisé des plantes de l'espèce *Raphanus sativus* porteuses d'une stérilité mâle cytoplasmique (dite stérilité Ogura) avec des plantes fertiles de l'espèce *Diplotaxis tenuifolia.* Ils ont effectué plusieurs rétro-croisements afin de revenir sur un noyau *Diplotaxis tenuifolia* tout en préservant la stérilité mâle cytoplasmique. A l'issue de ces différents croisements en retour, les plantes obtenues peuvent être utilisées comme parents femelles pour l'obtention d'hybrides.

Plus précisément, les inventeurs ont effectué des croisements entre des plantes de l'espèce *Raphanus sativus* comme parent femelle, porteuses de la stérilité mâle cytoplasmique dite Ogura, disponibles commercialement, avec des plantes de l'espèce *Diplotaxis tenuifolia* (fertiles) comme parent mâle. Ils ont ainsi obtenu neuf plantes F1 par sauvetage d'embryons, résultant de ce croisement intergénérique.

Dans une seconde étape, les inventeurs ont réalisé un premier rétro croisement (backcross 1 ou BC1) entre les plantes F1 obtenues (*Raphanus sativus* CMSx *Diplotaxis tenuifolia*) et des plantes de l'espèce *Diplotaxis tenuifolia,* c'est-à-dire le schéma suivant :

(*Raphanus sativus* CMS x *Diplotaxis tenuifolia*) x *Diplotaxis tenuifolia*

(en adoptant la convention internationale selon laquelle le parent femelle est mentionné du côté gauche de la croix et le parent mâle du côté droit).

Les conditions climatiques n'ont pas permis d'obtenir de plantes par sauvetage d'embryons, mais la répétition des mêmes croisements l'année suivante a conduit à l'obtention de six plantes qui étaient mâles stériles.

Les inventeurs ont ensuite procédé à un second rétro croisement (BC2) selon le schéma suivant :

((*Raphanus sativus* CMSx *Diplotaxis tenuifolia*) x *Diplotaxis tenuifolia*) x *Diplotaxis tenuifolia*.

Un embryon a été obtenu, par sauvetage d'embryons, et il a donné plusieurs plantes BC2 également mâle stériles.

Les étapes suivantes consistent en un troisième rétro croisement, toujours avec une plante de l'espèce *Diplotaxis tenuifolia,* suivi d'un quatrième rétro croisement (BC4).

A l'issue d'un quatrième rétro croisement, les plantes obtenues présentent l'ensemble des caractères phénotypiques caractérisant l'espèce *Diplotaxis tenuifolia* et peuvent être utilisés dans des programmes de sélection, sans qu'il soit nécessaire d'avoir recours au sauvetage d'embryons, notamment pour l'obtention de lignées cellulaires parentales porteuses d'une stérilité mâle cytoplasmique prêtes pour la production d'hydrides.

Les inventeurs ont par ailleurs réalisé plusieurs centaines de croisements *B. Oleracea* (stérilité mâle cytoplasmique Ogura) x *Diplotaxis tenuifolia* et *Brassica rapa* (stérilité mâle cytoplasmique Ogura) x *Diplotaxis tenuifolia,* mais aucun n'a permis d'obtenir d'embryons.

*Description plus détaillée des différentes étapes* :

1. Protocole pour les croisements intergénériques ou interspécifiques :

*Hybridation des fleurs :*

Aucune particularité, le protocole usuel décrit dans la littérature a été utilisé.

Les croisements ont été du type B. rapa x D. tenuifolia, B. oleracea x D. tenuifolia, R. sativus x D. tenuifolia , (R. sativus x D. tenuifolia) x D. tenuifolia, etc....

*Prélèvement des ovaires ou siliques :*

Les siliques sont prélevées avant qu'elles ne jaunissent sur la plante, pour mise en culture (entre 4 jours et un mois après le croisement). Sur les siliques prélevées, seules les siliques longues de plus de 20 mm avec un plus grand diamètre et des boursouflures sont utilisées.

*Désinfection* :

Les siliques sont mises dans des boules à thé. Elles sont passées à l'alcool pendant 5 minutes. Elles sont ensuite passées dans le bayrochlor 3cp/L d'eau stérile avec quelques gouttes de Tween 20 (polyoxyéthylène sorbitan monolaurate) pendant 30 minutes, en agitant régulièrement la boule à thé. Elles sont ensuite rincées trois fois de suite à l'eau stérile, en agitant de temps en temps la boule à thé.

*Mise en culture des ovules :*

Sur les siliques prélevées, seules celles ayant évoluées sont ouvertes. Ce sont les siliques de plus de 20 mm et avec un plus grand diamètre.

L'ouverture des siliques se fait le long de la fente de déhiscence, à l'aide d'une aiguille à dissocier lancéolée. Les ovules bien bombés et blanchâtres sont prélevés. Ils sont mis en culture de façon à ce que le hile soit en contact avec la gélose afin de permettre le développement de l'embryon.

Les ovules mis en culture sont observés régulièrement et repiqués tous les 15 jours. Les ovules qui noircissent et/ou qui deviennent plats (ce qui est synonyme d'avortement) sont éliminés.

*Conditions de culture pour les cultures d'ovaires et d'ovules*

Milieu N6, en lumière diffuse avec une photopériode de 16 heures, avec une température de 22°C durant les périodes de jour et 20°C durant les périodes de nuit. La composition de ce milieu N6 est détaillée dans les publications Chu, 1978 et Chu et al, 1975.

2. Développement et multiplication des plantules obtenues à partir des embryons.

*Composition des différents milieux de culture pour le développement et la multiplication des plantules obtenues :*

Le milieu mi 01 est un milieu de base de Murashige et Skoog (Murashige et Skoog, 1962) complémenté avec des vitamine de Morel B1, du saccharose et de l'Agar-Agar comme gélifiant. Le pH du milieu mi 01 est 5,8.

Le milieu mi 55 est un milieu de base de Murashige et Skoog (Murashige et Skoog, 1962), complémenté avec des vitamines de Morel, du saccharose et du Gelrite comme gélifiant, avec la substance de croissance $Ga_3$ après autoclavage. Le pH du milieu mi 55 est 5,9 / 6.

*Développement des embryons :*

Les embryons apparaissent entre 1 mois et 1 mois et demi après la mise en culture des ovules. Les embryons sont placés en lumière directe avec une photopériode de 16 heures, avec une température de 22°C durant les périodes de jour et 20°C durant les périodes de nuit.

Les embryons peuvent évoluer de deux façons : soit ils forment une plantule, soit il y a formation d'un cal, régénérant différentes plantules. En effet, les embryons mis en culture n'ont pas donné tout de suite des plantules, il y a eu formation de cals, avec parfois des bourgeons dessus.

Les plantules sont isolées sur un milieu de développement, le milieu 01 (ou Mi 01).

*Multiplication des explants*

Pour les cals :

Les cals sont repiqués sur un milieu d'induction N6 ou Mi55, pour régénérer des bourgeons. Les bourgeons sont repiqués sur le milieu Mi 01.

Pour les bourgeons :

Tous les bourgeons issus des cals ont subi des subcultures sur le milieu de développement Mi 01, pour s'enraciner.

Récapitulatif : pour le développement et la multiplication des explants, deux cas de figure sont possibles :

1) Il y a un passage par cal. La culture de cals s'effectue sur un milieu d'induction. Les cals régénèrent des bourgeons.
2) On dispose de bourgeons qui peuvent être directement développés. Cette culture s'effectue sur un milieu de développement Mi 01.

Afin de multiplier ces plantules, si la plantule n'a pas de problème de développement, la culture de noeuds est utilisée sur milieu Mi 01. Ces noeuds (ou bourgeons axillaires) se développent en plantules.

Si la plantule est chétive et/ou a des problèmes de développement, elle est repiquée sur un milieu de multiplication Mi 55.

*Enracinement des plantules* :

Pour enraciner les plantules multipliées, en vue d'une acclimatation, ces dernières sont cultivées sur un milieu d'enracinement Mi 01.

*Acclimatation :*

Les inventeurs ont ensuite procédé à l'acclimatation dès que les plantules avaient des racines bien développées. La culture *in vitro* a été poursuivie jusqu'à l'obtention de telles racines.

Les plantes sont acclimatées en godets individuels avec un substrat horticole Klasmann Potgrong H, et maintenues sous mini serre pendant une semaine. Au bout d'une semaine, les inventeurs ont obtenu de belles plantes avec un bon enracinement, qui ont été sorties des mini serres.

Au bout de trois semaines, les plantes présentaient un bon système racinaire et un bon développement foliaire. Les plantes ont été ensuite plantées en pleine terre, sous tunnel. Le phénotypage des plantes ainsi obtenues est réalisé afin de sélectionner celles dont les caractéristiques phénotypiques sont les plus proches de *D. tenuifolia,* tout en étant porteuses de la stérilité mâle cytoplasmique (détectable phénotypiquement du fait de l'absence de pollen).

3. Résultats

Croisements intergénériques

- Les inventeurs ont réalisés les croisements intergénériques suivants :
  *Brassica rapa* CMS x *Diplotaxis tenuifolia* et *B. oleracea CMS* x *Diplotaxis tenuifolia,* suivis des premiers rétro croisements suivants :
  *(Brassica rapa* CMS x *Diplotaxis tenuifolia)* x *Diplotaxis tenuifolia* et *(B. oleracea CMS* x *Diplotaxis tenuifolia)* x *Diplotaxis tenuifolia.*

Pour ces deux rétro-croisements, aucun embryon n'a pu être obtenu, et donc aucune descendance.

- Les inventeurs ont alors procédé au croisement intergénérique suivant :

*Raphanus sativus* (CMS) x *Diplotaxis tenuifolia*.

Les siliques ont été prélevées et les ovaires ont été mis en culture. Au total, 135 hybridations *Raphanus sativus* (CMS) x *Diplotaxis tenuifolia* ont été réalisées, 1857 siliques ont été prélevées et 207 ovules mis en culture ; par cette technique, 9 embryons ont été obtenus, codifiés RD1 à RD9.

Des plantes ont ainsi été obtenues *in vitro,* qui ont ensuite été acclimatées.

Premier croisement en retour :

L'année suivante, les inventeurs ont réalisé le premier croisement en retour, selon le schéma : *[Raphanus sativus* (CMS) x *Diplotaxis tenuifolia* ]x *Diplotaxis tenuifolia.*

Le tableau 1 récapitule le nombre de siliques prélevées avec ovules.

Tableau n°1 :

| Code parent femelle | Nb de siliques prélevées | Nb de siliques avec ovules | % de siliques avec ovules | Nb d'ovules mis en culture |
|---|---|---|---|---|
| RD1 | 49 | 22 | 44,9% | 400 |
| RD1 | 100 | 42 | 42% | 635 |
| RD2 | 0 | 0 | | 0 |
| RD2 | 0 | 0 | | 0 |
| RD4 | 12 | 8 | 66,7% | 161 |
| RD4 | 55 | 32 | 58,2% | 561 |
| RD5 | 38 | 2 | 5,3% | 2 |
| RD8 | 10 | 3 | 30% | 4 |
| RD8 | 26 | 1 | 3,8% | 2 |
| RD8 | 10 | 2 | 20% | 2 |
| RD8 | 15 | 6 | 40% | 13 |
| RD9 | 38 | 14 | 36,8% | 224 |
| RD9 | 72 | 34 | 47,2% | 887 |
| **total** | **425** | **166** | **39,1%** | **2891** |

Le taux de siliques contenant des ovules est moyen (fréquence de 39,1% en moyenne).

Les ovules ont été repiqués deux fois sur du milieu neuf N6. Aucun embryon BC1 n'a été obtenu pour les 2891 ovules mis en culture. Il est toutefois à noter que les premiers croisements ont été réalisés dans de mauvaises conditions climatiques.

Les inventeurs ont alors réalisé un nouvel essai de rétro croisement l'année suivante, en modifiant la méthode de fécondation par rapport à l'année précédente. En effet, il a été décidé de ne pas effectuer toutes les fécondations simultanément mais de les étaler sur un mois, afin de limiter l'influence des conditions climatiques, et de réaliser au plus 200 boutons à chaque jour de fécondation.

Le tableau 2 récapitule les résultats de cette deuxième tentative de rétro-croisement.

| Code parent femelle | Nb de siliques prélevées | Nb d'ovules mis en culture | Nb d'ovules ayant germé | Nb embryon obtenu | Code plantes obtenues |
|---|---|---|---|---|---|
| RD8 | 24 | 58 | 0 | | |
| RD8 | 6 | 0 | 0 | | |
| RD9 | 50 | 205 | 1 | 0 | 0 |
| RD3 | 6 | 0 | 0 | | |
| RD3 | 0 | 0 | 0 | | |
| RD6 | 0 | 0 | 0 | | |
| RD6 | 3 | 4 | 0 | | |
| RD8 | 36 | 0 | 0 | | |
| RD8 | 24 | 2 | 1 | | |
| RD2 | 40 | 0 | 0 | | |

(suite)

| Code parent femelle | Nb de siliques prélevées | Nb d'ovules mis en culture | Nb d'ovules ayant germé | Nb embryon obtenu | Code plantes obtenues |
|---|---|---|---|---|---|
| RD8 | 33 | 10 | 0 | | |
| RD9 | 21 | 80 | 0 | | |
| RD3 | 0 | 0 | 0 | | |
| RD8 | 25 | 11 | 4 | 2 | D6 - D7 |
| RD9 | 38 | 168 | 0 | | |
| RD2 | 0 | 0 | 0 | | |
| RD3 | 0 | 0 | 0 | | |
| RD8 | 31 | 80 | 0 | | |
| RD8 | 32 | 7 | 0 | | |
| RD8 | 21 | 73 | 0 | | |
| RD9 | 15 | 140 | 0 | | |
| RD9 | 24 | 61 | 0 | | |
| RD8 | 23 | 50 | 0 | | |
| RD8 | 0 | 0 | 0 | | |
| RD8 | 56 | 130 | 0 | | |
| RD8 | 32 | 119 | 0 | | |
| RD9 | 27 | 100 | 1 | 1 | D4 |
| RD8 | 58 | 111 | 0 | | |
| RD8 | 27 | 141 | 0 | | |
| RD8 | 14 | 19 | 0 | | |
| RD8 | 21 | 380 | 3 | 4 | D1-D2-D3-D5 |
| RD3 | 7 | 0 | 0 | | |
| RD8 | 26 | 8 | 0 | | |
| **total** | **720** | **1957** | **10** | **7** | |

Comme il ressort de ce tableau 2, 7 embryons ont été obtenus au total, dénommés D1 à D7. La plantule D7 ne s'est pas développée.

Le repiquage des embryons sur milieu N6 permet la prolifération des bourgeons : chaque embryon peut être ainsi cloné en plusieurs exemplaires. Il est ainsi possible d'acclimater des plantes qui servent ensuite aux rétro-croisements suivants, tout en maintenant *in vitro* des plantules.

Le matériel F1 et BC1 sont également entretenus régulièrement *in vitro,* ou bien en serre (lorsque des problèmes de contamination apparaissent).

Les plantes acclimatées sont remises en culture à partir d'axillaires. Les tronçons de tiges sont désinfectés avec une solution de Bayrochlor (3 cp/L) pendant 30 minutes. Les axillaires sont déposés sur un milieu de base (par exemple Mi01).

<u>Second croisement en retour :</u>

Les inventeurs ont ensuite réalisé le second croisement en retour, selon le schéma :

*[[Raphanus sativus* (CMS) x *Diplotaxis tenuifolia* ]x *Diplotaxis tenuifolia ]* x *Diplotaxis tenuifolia*

Les siliques sont prélevées quelques jours après hybridation, pour mise en culture des ovules.

Avec les 60 hybridations réalisées, 1149 siliques ont été prélevées et 5505 ovules mis en cultures.

Un unique embryon a été obtenu, il a été repiqué tous les 15 jours sur le milieu mi 55 ou N6 pour obtenir un cal, puis des bourgeons qui se sont développés en plantules.

**Exemple 2 : Caractérisation du génome mitochondrial et chloroplastique de plantes *Diplotaxis tenuifolia.***

Le génome chloroplastique et le génome mitochondrial se caractérisent par une hérédité exclusivement maternelle chez les Brassicaceae. Les inventeurs ont utilisé trois gènes mitochondriaux décrits dans le brevet EP 0 549 726 et des marqueurs microsatellites (ou SSR pour « simple sequence repeat ») chloroplastiques décrits dans la publication Flannery et al (2006), afin de caractériser le cytoplasme mâle chez des plantes *Diplotaxis tenuifolia* mâle stérile selon l'invention.

En effet, il est rappelé qu'une plante *Diplotaxis tenuifolia* selon l'invention comprend dans son génome mitochondrial et dans son génome chloroplastique des séquences provenant de *Raphanus sativus.*

Les oligonucleotides définis au sein du gène orf138 et du gène de l'ARNt de transfert formylméthionine permettent, respectivement, l'amplification d'un fragment de 512 et 401 paires de bases, uniquement dans les plantes qui comprennent la stérilité Ogura. L'amplification au sein du gène cox I (petite sous-unité 1 de la cytochrome oxydase) d'un fragment du génome mitochondrial de radis de 655 paires de bases, sert de contrôle, dans la mesure où ce fragment doit être amplifié dans toutes les lignées testées.

En fonction de la présence ou de l'absence d'amplification de fragments de séquences mitochondriales à l'aide de ces amorces, il est possible de caractériser les différentes plantes utilisées dans la présente invention quant à leur stérilité mâle cytoplasmique.

La variation allélique des marqueurs microsatellites SSR chloroplastiques testés permet de caractériser la provenance du génome chloroplastique des plantes *Diplotaxis tenuifolia* de l'invention, plus particulièrement la présence de séquences chloroplastiques caractéristiques de *Raphanus sativus.*

En fonction de la longueur du fragment d'amplification de séquences chloroplastiques à l'aide de ces amorces, il est possible de caractériser les différentes plantes utilisées dans la présente invention quant à la nature de leur génome chloroplastique.

Les plantes *Diplotaxis tenuifolia* obtenues selon le procédé décrit dans cette invention se distinguent au niveau de leur génome chloroplastique, d'autres plantes *Diplotaxis tenuifolia* dont le génome chloroplastique n'est pas originaire du genre Raphanus.

**A. Analyse des marqueurs mitochondriaux et conditions de PCR.**

L'ADN total (génomes nucléaires et cytoplasmiques) de plantes *D. tenuifolia* fertiles et mâle-stériles selon l'invention, a été isolé à partir des feuilles de plantes âgées de 8 semaines, selon le protocole de Dellaporta (1983). - Utilisation de marqueurs spécifiques de orf138 et T-ARNt afin de détecter la stérilité mâle cytoplasmique Ogura :

Orf138

Amorce 'sens' : oligo 37 : 5'-GCATCACTCTCCCTGTCGTTATCG-3' (8μM) (SEQ ID N°22) ;
Amorce 'antisens' : oligo38 : 5'-ATTATTTTCTCGGTCCATTTTCCA-3' (8μM) (SEQ ID N°23).

T-ARNt

Amorce 'sens': TRNAFM-610U : 5'-ACGTGTAGCCCTGTATGGACT-3' (8μM) (SEQ ID N°24) ;
Amorce 'antisens': TRNAFM-987L : 5'-GGTATTGTCACTTCCCGTTTC-3' (8μM) (SEQ ID N°25).

La position de ces différentes amorces est illustrée sur la figure 1 (soulignement pour les amorces liées à T-ARNt et double soulignement pour les amorces liées à orf138).

- Utilisation de marqueurs spécifiques pour amplifier un contrôle mitochondrial positif dans toutes les plantes testées :

Amorce 'sens': COX1-244U : 5'-GGTAATTGGTTTGTTCCGATT-3' (8μM) (SEQ ID N°26) ;
Amorce 'antisens': COX1-805L : 5'-CATGCCTAGATACCCGAAGAC-3' (8μM) (SEQ ID N°27).

- La réaction PCR pour ces marqueurs est réalisée sur des échantillons de 20μl comprenant :

5.0 µl de l'ADN total dilué (2 à 10 ng/µl)
2.0 µl du tampon PCR 10 x (Invitrogen)
2.0 µl de $MgCl_2$ 25 mM (Invitrogen)
1,5 µl de mélange de dNTP (2mM de chaque dNTP; SIGMA)
0.7 µl Amorce 'sens' (8µM)
0.7 µl Amorce 'antisens' (8µM)
0.15 µl ADN polymerase Taq 5U/µl (Invitrogen)
7.95 µl $H_2O$.

Le profil des thermocycles de PCR est le suivant :

- pour l'amplification de ORF138 et T-ARNt
  3 minutes à 94°C; puis 35 cycles de (30 sec à 94°C, 45 sec à 68,5°C, 1 min à 72°C); 7 min à 72°C puis retour à 4°C, sur un système PCT GeneAmp® 2700 (Applied Biosystems).
- pour l'amplification de COX1 :
  3 minutes à 94°C; puis 35 cycles de (30 sec à 94°C, 45 sec à 54°C, 1 min à 72°C); 7 min à 72°C puis retour à 4°C, sur un système PCT GeneAmp® 2700 (Applied Biosystems).

Une partie des produits PCR est vérifiée sur un gel d'agarose à 1,5% et le restant est soumis à séquençage selon la méthode de Sanger.

La figure 2 montre une bande correspondant à l'amplification de COX1 pour tous les échantillons. Les bandes d'amplification représentant les fragments de ORF138 et T-ARNt ne sont quant à elles présentes que dans les colonnes chargées avec de l'ADN mitochondrial de plantes porteuses de la stérilité mâle cytoplasmique Ogura.

L'échelle de taille présente dans la partie la plus à gauche du gel confirme que les fragments amplifiés sont de taille conforme aux prédictions, i.e. environ 512 paires de bases pour ORF138, environ 401 pour T-ARNt et environ 655 pour COX1.

Les inventeurs ont également réalisé un séquençage de deux fragments amplifiés (ORF138 et T-ARNt), qui a permis de confirmer que leur taille était conforme aux tailles attendues, mais également que la séquence des fragments amplifiés était identique ou quasiment identique à la séquence répertoriée comme étant liée à la CMS Ogura, chez Raphanus sativus, à savoir Z18896.

Le séquençage des fragments amplifiés (consensus du séquençage de plusieurs fragments) a donné les résultats suivants (les séquences soulignées correspondent aux séquences des amorces) :

- Fragment amplifié de 512 paires de bases de l'orf138 :

GCATCACTCTCCCTGTCGTTATCGACCTCGCAAGGTTTTTGAGACGGCCGAAACGGGAAGTGACAA
TACCGCTTTTCTTCAGCATATAAATGCAATGATTACCTTTTTCGAAAAATTGTCCACTTTTTGTCA
TAATCTCACTCCTACTGAATGTAAAGTTAGTGTAATAAGTTTCTTTCTTTTAGCTTTTTTACTAAT

GGCCCATATTTGGCTAAGCTGGTTTTCTAACAACCAACATTGTTTACGAACCATGAGACATCTAGA
GAAGTTAAAAATTCCATATGAATTTCAGTATGGGTGGCTAGGTGTCAAAATTACAATAAAATCAAA
TGTACCTAACGATGAAGTGACGAAAAAAGTCTCACCTATCATTAAAGGGGAAATAGAGGGGAAAGA
GGAAAAAAAAGAGGGGAAAGGGGAAATAGAGGGGAAAGAGGAAAAAAAAGAGGGGAAAGGGGAAAT
AGAGGGGAAAGAGGAAAAAAAAGAGGTGGAAAATGGACCGAGAAAATAAT (SEQ ID N°20).

Une seule divergence d'un nucléotide (position 43 de SEQ ID N°20) a été relevée par rapport à la séquence correspondante de Z18896 (SEQ ID N°1).

- Fragment amplifié de 401 paires de bases du T-ARNt :

ACGTGTAGCCCTGTATGGACTCGCGAAGCAGGTCTCCGGTCGGTGTCCAAGATTTGATCTAACTAT
TGAGTGAGGACTACTTACCGATTGATAGAATAATACGTATATAAGAAGAAGGCTGCTTTGTGGAGT
GATCTTTCTCGAAATGAATTAAGTAAGGGCGCTATGTTCAGATTCTGAACCAAAGCACTAGTTGAG
GTCTGAAAGCCTTATGAGCAGAAGTAATAAATACCTCGGGGAAGAAGCGGGGTAGAGGAATTGGTC
AACTCATCAGGCTCATGACCTGAAGATTACAGGTTCAAATCCTGTCCCCGCACCGTAGTTTCATTC
TGCATCACTCTCCCTGTCGTTATCGACCTCGCAAGGTTTTTGAAACGGCCGAAACGGGAAGTGACA
ATACC (SEQ ID N°21).

Aucune divergence n'a été relevée entre SEQ ID N°21 et la séquence correspondante de Z18896 (SEQ ID N°1).

Ceci permet de conclure que le génome mitochondrial des plantes *Diplotaxis tenuifolia* mâle stériles selon la présente invention est bien celui du parent femelle dans le croisement intergénérique, c'est-à-dire celui de *Raphanus sativus* CMS.

**B. Analyse des marqueurs chloroplastiques et conditions de PCR.**

- Utilisation de SSR (répétion d'une séquence simple ou marqueur microsatellite) pour distinguer entre des plantes fertiles et des plantes porteuses de la stérilité mâle cytoplasmique selon l'invention.

Les marqueurs utilisés sont répertoriés dans le tableau 3.

L'analyse des polymorphismes des marqueurs SSR est réalisée sur un système d'analyse ADN MegaBACE®, à l'aide d'une matrice de séparation LPA haute résolution, avec une résolution d'une unique base (General Electrics Healthcare Inc).

Ces marqueurs SSR sont amplifiés en utilisant la queue M13 universelle qui permet l'amplification de nombreux fragments SSR ciblés à l'aide d'une unique amorce M13 marquée (différents colorants disponibles tels que FAM, HEX ou NED) et de nombreuses amorces 'sens'-M13.

- Le milieu réactionnel pour la PCR consiste en un échantillon de 10μL comprenant :

5.0 μl de l'ADN total dilué (2 à 10 ng/μl)
1.0 μl du tampon PCR10 x (Invitrogen)
1.0 μl $MgCl_2$ 25mM (Invitrogen)
1.0 μl de mélange de dNTP (2mM de chaque dNTP; SIGMA)
0.6 μl Amorce 'sens'-M13 (2μM)
0.6 μl Amorce 'antisens' (8μM)
0.12 μl Amorce M13 marquées avec FAM, HEX ou NED (8μM)
0.12 μl ADN polymerase Taq 5U/μl (Invitrogen)
0.56 μl $H_2O$.

Le profil des thermocycles de PCR est le suivant :
3 minutes à 94°C; puis 35 cycles de (15 sec à 94°C, 20 sec à 50°C, 15 sec à 72°C); 7 min à 72°C puis retour à 4°C, sur un système PCT GeneAmp® 2700 (Applied Biosystems). L'analyse de génotypage est réalisée sur MegaBACE®.

Quatre à six produits SSR sont dilués au vingtième et multiplexé dans le même capillaire avec 5 μl de ET-400 ROX (échelle standard pour la taille). Les conditions d'utilisation sont les suivantes :

Temps d'injection : 110 secondes ;
Voltage à l'injection : 3 kV ;
Temps de parcours : 65 minutes
Voltage durant le parcous : 9kV
Filtres : set de colorants II

Le programme MegaBACE® Fragment Profiler 1.0 permet de convertir les données brutes en données relatives à la taille. Le tableau 4 contient les allèles SSR obtenus sur différentes plantes fertiles et mâle-stériles selon la présente invention.

L'analyse a été effectuée sur 2 lignées différentes de *Diplotaxis tenuifolia* fertiles et sur 2 lignées de *Diplotaxis tenuifolia* selon la présente invention, ainsi que sur une lignée de *B. oleracea* porteuse de la stérilité mâle cytoplasmique Ogura dite selon le brevet EP0 549 726, et sur une lignée de *R. sativus* porteuse de la stérilité mâle cytoplasmique Ogura.

**Tableau 3** : amorces utilisées pour le génotypage chloroplastique.

| **amorce** | **séquence** | **SEQ ID** | **gène** |
|---|---|---|---|
| MF1_M13F | 5'-CACGACGTTGTAAAACGACTCAATTGCACATTCTAGAATTCTAAG-3' | SEQ ID N°2 | **Gène trnL-F** |
| MF1_R | 5'-CAATTCAATATGGTTATATATTAGAG-3' | SEQ ID N°3 | |
| MF2_M13F | 5'-CACGACGTTGTAAAACGACGGTTCCGTCGTTCCCATCGC-3' | SEQ ID N°4 | **Gène RPL16** |
| MF2_R | 5'-CATAATAATTAGATAAATCTGTTCC-3' | SEQ ID N°5 | |
| MF3_M13F | 5'-CACGACGTTGTAAAACGACAATGGTATGACTAGCTTATAAGG-3' | SEQ ID N°6 | **Gène trnE-trnT** |
| MF3_R | 5'-CTTAACAATGAGATGAGGCAATC-3' | SEQ ID N°7 | |
| MF4_M13F | 5'-CACGACGTTGTAAAACGACCGGATCTATTATGACATATCC-3' | SEQ ID N°8 | **Gène psaA-ycf3** |
| MF4_R | 5'-GAAATATGAATACACTAGATTAGG-3' | SEQ ID N°9 | |
| MF5_M13F | 5'-CACGACGTTGTAAAACGACCCTGGCGGTATCAAGATGCCACT-3' | SEQ ID N°10 | **Gène trnT-rpoC2** |
| MF5_R | 5'-GCCATAATGGTACAGAACTAT-3' | SEQ ID N°11 | |
| MF6_M13F | 5'-CACGACGTTGTAAAACGACGAAGGAATAGTCGTTTTCAAG-3' | SEQ ID N°12 | **Gène atpB-rbcL** |
| MF6_R | 5'-CATAATTAGAGTTCCATTTCGG-3' | SEQ ID N°13 | |
| MF7_M13F | 5'-CACGACGTTGTAAAACGACCGGCAGGAGTCATTGGTTCAAA-3' | SEQ ID N°14 | **Gène TrnM-atpE** |
| MF7_R | 5'-GATTTTGTAACTAGCTGACG-3' | SEQ ID N°15 | |
| MF8_M13F | 5'-CACGACGTTGTAAAACGACCTTATATTCATAAGCGAAGAAC-3' | SEQ ID N°16 | **Gène rbcL-accD** |
| MF8_R | 5'-AATAACAATAGATGAATAGTCA-3' | SEQ ID N°17 | |
| MF9_M13F | 5'-CACGACGTTGTAAAACGACGGGCCGTTATGCTCATTACG-3' | SEQ ID N°18 | **Gène ndhB-rps7** |
| MF9_R | 5'-TCCTATTCATGGGGATTCCG-3' | SEQ ID N°19 | |

**Tableau 4 :** résultats du génotypage avec 9 SSR chloroplastiques. Les nombres représentent la taille des allèles des fragments d'amplification en nombre de bases.

| | MF1 | MF2 | MF8 | MF7 | MF9 | MF4 | MF3 | MF5 | MF6 |
|---|---|---|---|---|---|---|---|---|---|
| B. oleracea CMS (brevet EP 0549 726) | 184 | 190 | 265 | 170 | 328 | 160 | 306 | 218 | 178 |
| ***Raphanus sativus CMS Ogura*** | **183** | **193** | **264** | **180** | **319** | **158** | **303** | **218** | **183** |

(suite)

| | MF1 | MF2 | MF8 | MF7 | MF9 | MF4 | MF3 | MF5 | MF6 |
|---|---|---|---|---|---|---|---|---|---|
| D. tenuifolia 1 | 183 | 190 | 146 | 173 | 327 | 164 | 298 | 218 | 176 |
| D. tenuifolia 3 | 183 | 191 | 147 | 173 | 327 | 158 | 312 | 218 | 180 |
| **CMS D. tenuifolia 1** | **183** | **193** | **264** | **180** | **319** | **158** | **303** | **218** | **183** |
| **CMS D. tenuifolia 2** | **183** | **193** | **264** | **180** | **319** | **158** | **303** | **218** | **183** |

Il est rappelé que les chloroplastes d'une plante *Diplotaxis tenuifolia* selon l'invention proviennent de *Raphanus sativus.*

Dans des plantes de type *B. oleracea* porteuses de la stérilité mâle cytoplasmique Ogura, suite à la fusion de protoplases qui a permis d'importer la stérilité mâle cytoplasmique de R. sativus à B. oleracea, les chloroplastes se distinguent de ceux de R. sativus.

Les résultats du tableau 4 confirment que les longueurs des fragments amplifiés sont identiques pour les 2 lignées de *Diplotaxis tenuifolia* selon la présente invention (c'est-à-dire CMS, les deux dernières lignes du tableau), ces longueurs sont également identiques à celles des fragments amplifiés pour la lignée de *Raphanus sativus* mâle-stérile Ogura. Ceci permet de conclure que le génome chloroplastique des plantes *Diplotaxis tenuifolia* mâle stériles selon la présente invention est bien originaire de *Raphanus sativus* (utilisée comme parent femelle dans le croisement intergénérique).

Les résultats obtenus relatifs au génome chloroplastique confirment que le génome chloroplastique des plantes de l'invention correspond au génome chloroplastique de *Raphanus sativus,* et qu'il se distingue du génome chloroplastique des plantes telles que décrites dans le brevet EP0 549 726.

Par ailleurs, le génome chloroplastiques de plantes selon l'invention, porteuses de la stérilité mâle cytoplasmique, se distingue également de celui de plantes de l'espèce *Diplotaxis tenuifolia* mâles fertiles.

Aux fins de distinction entre une plante *D. tenuifolia* mâle stérile selon la présente invention et une plante *D. tenuifolia* fertile, les couples de marqueurs suivants sont particulièrement adaptés :

- les amorces oligo MF2_M13F et MF2_R (respectivement SEQ ID N°4 et 5) qui permettent d'obtenir un fragment d'amplification de 193 paires de bases, caractéristique du génome chloroplastique de *Raphanus sativus ;*
- les amorces oligo MF8_M13F et MF8_R (respectivement SEQ ID N°16 et 17) qui permettent d'obtenir un fragment d'amplification de 264 paires de bases, caractéristique du génome chloroplastique de *Raphanus sativus ;*
- les amorces oligo MF8 MF7_M13F et MF7_R (respectivement SEQ ID N°14 et 15) qui permettent d'obtenir un fragment d'amplification de 180 paires de bases, caractéristique du génome chloroplastique de *Raphanus sativus ;*
- les amorces oligo MF9_M13F et MF9_R (respectivement SEQ ID N°18 et 19) qui permettent d'obtenir un fragment d'amplification de 319 paires de bases, caractéristique du génome chloroplastique de *Raphanus sativus ;*
- les amorces oligo MF3_M13F et MF3_R (respectivement SEQ ID N°6 et 7) qui permettent d'obtenir un fragment d'amplification de 303 paires de bases, caractéristique du génome chloroplastique de *Raphanus sativus ;*
- les amorces oligo MF6_M13F et MF6_R (respectivement SEQ ID N°12 et 13) qui permettent d'obtenir un fragment d'amplification de 183 paires de bases, caractéristique du génome chloroplastique de *Raphanus sativus ;*
- les amorces oligo 37 et 38 (respectivement SEQ ID N°22 et 23) qui permettent d'obtenir un fragment d'amplification de 512 paires de bases, caractéristique du génome mitochondrial de *Raphanus sativus CMS (Ogura);*
- les amorces TRNAFM-610U et TRNAFM-987L (respectivement SEQ ID N°24 et 25) qui permettent d'obtenir un fragment d'amplification de 401 paires de bases, caractéristique du génome mitochondrial de *Raphanus sativus CMS (Ogura).*

**Bibliographie** :

Cao J. et Earle, E.D. Plant Cell Report 21 :789-796 (2003).

Chu C.C The N- medium and its application to anther culture of Cereal crops Proc. Symp. Plant Tissue culture, Peking 43 (1978).

Chu C.C et al Establishement of an efficient medium for anther culture of Rice through comparative experiments on the nitrogen sources, Scienta Sinic, 18 , 659 (1975). Flannery M.L. et al. Plastid genome characterisation in Brassica and Brassicaceae using a new set of nine SSRs. Theor Appl Genet 113:1221-1231 (2006).

Matsuzawa et al. Male sterility in alloplasmic Brassica rapa L. carrying Eruca sativa cytoplasm. Plant Breeding 118, 82-84 (1999).

Murashige, T. and Skoog F. A revised medium for rapid growth and bioassays with tobacco tissue cultures.

Physiol. Plant 15, 473 (1962)
Rahman, M.H. Optimum age of siliques for rescue of hybrid embryos from crosses between Brassica oleracea, B. rapa and B. carinata. Canadian Journal of Plant Science 84 (4) : 965-969 (2004).
Woo Bang, S. et al. (2003). Production of Intergeneric Hybrids between the C3-C4 Intermediate Species Diplotaxis tenuifolia (L.) DC. and Raphanus sativus L. Breeding Sciences 53: 231-236

**Revendications**

1. Plante de l'espèce *Diplotaxis tenuifolia,* **caractérisée en ce qu'**elle est porteuse d'une stérilité mâle cytoplasmique, **en ce que** son génome mitochondrial comprend la séquence d'ADN correspondant aux nucléotides 1673 à 2089 de SEQ ID N°1, ou une séquence ayant au moins 70% d'identité avec cette séquence d'ADN, et **en ce qu'**elle ne présente aucun symptôme de chlorose en conditions normales de culture, notamment pas de décoloration des feuilles due à un manque de chlorophylle.

2. Plante selon la revendication 1, **caractérisée en ce qu'**il s'agit d'une plante hybride.

3. Plante selon la revendication 1, **caractérisée en ce qu'**elle comprend dans son génome cytoplasmique chloroplastique une séquence d'au moins 200 paires de bases consécutives, provenant du génome chloroplastique de *Raphanus sativus.*

4. Plante selon la revendication 3, **caractérisée en ce qu'**on obtient un fragment d'amplification de la taille suivante quand les amorces correspondantes sont utilisées pour amplifier par PCR son génome chloroplastique :

   • 193 paires de bases quand les amorces SEQ ID N°4 et 5 sont utilisées et/ou
   • 264 paires de bases quand les amorces SEQ ID N°16 et 17 sont utilisées et/ou
   • 180 paires de bases quand les amorces SEQ ID N°14 et 15 sont utilisées et/ou
   • 319 paires de bases quand les amorces SEQ ID N°18 et 19 sont utilisées et/ou
   • 303 paires de bases quand les amorces SEQ ID N°6 et 7 sont utilisées et/ou
   • 183 paires de bases quand les amorces SEQ ID N°12 et 13 sont utilisées.

5. Plante selon la revendication 1, **caractérisée en ce qu'**on obtient un fragment d'amplification de la taille suivante quand les amorces correspondantes sont utilisées pour amplifier par PCR son génome mitochondrial :

   • 512 paires de bases quand les amorces SEQ ID N°22 et 23 sont utilisées et /ou
   • 401 paires de bases quand les amorces SEQ ID N°24 et 25 sont utilisées.

6. Plante selon l'une des revendications 1 à 5, **caractérisée en ce que** ses cellules possèdent l'ADN génomique de *Diplotaxis tenuifolia* et l'ADN mitochondrial et chloroplastique de *Raphanus sativus.*

7. Graine de l'espèce *Diplotaxis tenuifolia,* **caractérisée en ce qu'**elle est porteuse d'une stérilité mâle cytoplasmique, et **en ce que** son génome mitochondrial comprend la séquence d'ADN correspondant aux nucléotides 1673 à 2089 de SEQ ID N°1, ou une séquence ayant au moins 70% d'identité avec cette séquence d'ADN.

8. Graine selon la revendication 8, **caractérisée en ce qu'**elle comprend dans son génome chloroplastique des séquences provenant de *Raphanus sativus,* d'au moins 200 paires de bases consécutives.

9. Cellule d'une plante de l'espèce *Diplotaxis tenuifolia* **caractérisée en ce qu'**elle est porteuse d'une stérilité mâle cytoplasmique et **en ce que** son génome mitochondrial comprend la séquence d'ADN correspondant aux nucléotides 1673 à 2089 de SEQ ID N°1, ou une séquence ayant au moins 70% d'identité avec cette séquence d'ADN.

10. Cellule selon la revendication 9, issue d'une plante selon l'une quelconque des revendications 1 à 6 ou bien d'une graine selon l'une quelconque des revendications 7 à 8.

11. Cellule selon la revendication 10, **caractérisée en ce qu'**elle comprend dans son génome chloroplastique des séquences provenant de *Raphanus sativus,* d'au moins 200 paires de bases consécutives.

## Patentansprüche

1. Pflanze der Art *Diplotaxis tenuifolia,* **dadurch gekennzeichnet, dass** sie Trägerin einer cytoplasmatischen männlichen Sterilität ist, dass ihr Mitochondriengenom die den Nukleotiden 1673 bis 2089 der SEQ ID Nr. 1 entsprechende DNA-Sequenz umfasst, oder eine Sequenz, die mit dieser DNA-Sequenz zumindest zu 70 % identisch ist, und dass sie unter normalen Kulturbedingungen keine Chlorosesymptome aufweist, insbesondere keine Entfärbung der Blätter wegen Chlorophyllmangels.

2. Pflanze nach Anspruch 1, **dadurch gekennzeichnet, dass** es sich um eine Hybridpflanze handelt.

3. Pflanze nach Anspruch 1, **dadurch gekennzeichnet, dass** sie in ihrem cytoplasmatischen Chloroplastengenom eine Sequenz von mindestens 200 aufeinanderfolgenden Basenpaaren umfasst, die aus dem Chloroplastengenom von *Raphanus sativus* stammen.

4. Pflanze nach Anspruch 3, **dadurch gekennzeichnet, dass** man ein Amplifikationsfragment der nächsten Größe erhält, wenn die entsprechenden Primer zur PCR-Amplifikation ihres Chloroplastengenoms verwendet werden:

   - 193 Basenpaare, wenn die Primer SEQ ID Nr. 4 und 5 verwendet werden und/oder
   - 264 Basenpaare, wenn die Primer SEQ ID Nr. 16 und 17 verwendet werden und/oder
   - 180 Basenpaare, wenn die Primer SEQ ID Nr. 14 und 15 verwendet werden und/oder
   - 319 Basenpaare, wenn die Primer SEQ ID Nr. 18 und 19 verwendet werden und/oder
   - 303 Basenpaare, wenn die Primer SEQ ID Nr. 6 und 7 verwendet werden und/oder
   - 183 Basenpaare, wenn die Primer SEQ ID Nr. 12 und 13 verwendet werden.

5. Pflanze nach Anspruch 1, **dadurch gekennzeichnet, dass** man ein Amplifikationsfragment der nächsten Größe erhält, wenn die entsprechenden Primer zur PCR-Amplifikation ihres Mitochondriengenoms verwendet werden:

   - 512 Basenpaare, wenn die Primer SEQ ID Nr. 22 und 23 verwendet werden und /oder
   - 401 Basenpaare, wenn die Primer SEQ ID Nr. 24 und 25 verwendet werden.

6. Pflanze nach einem der Ansprüche 1 bis 5, **dadurch gekennzeichnet, dass** ihre Zellen die genomische DNA von *Diplotaxis tenuifolia* und die mitochondriale und chloroplastische DNA von *Raphanus sativus* besitzen.

7. Samen der Art *Diplotaxis tenuifolia,* **dadurch gekennzeichnet, dass** er Träger einer cytoplasmatischen männlichen Sterilität ist und sein Mitochondriengenom die den Nukleotiden 1673 bis 2089 der SEQ ID Nr. 1 entsprechende DNA-Sequenz umfasst, oder eine Sequenz, die zumindest zu 70 % mit dieser DNA-Sequenz identisch ist.

8. Samen nach Anspruch 8, **dadurch gekennzeichnet, dass** er in seinem Chloroplastengenom Sequenzen von mindestens 200 aufeinanderfolgenden Basenpaaren umfasst, die von *Raphanus sativus* stammen.

9. Zelle einer Pflanze der Art *Diplotaxis tenuifolia,* **dadurch gekennzeichnet, dass** sie Trägerin einer cytoplasmatischen männlichen Sterilität ist und ihr Mitochondriengenom die den Nukleotiden 1673 bis 2089 der SEQ ID Nr. 1 entsprechende DNA-Sequenz umfasst oder eine Sequenz, die mit dieser DNA-Sequenz zumindest zu 70 % identisch ist.

10. Zelle nach Anspruch 9, die aus einer Pflanze nach einem der Ansprüche 1 bis 6 oder aus einem Samen nach einem der Ansprüche 7 bis 8 stammt.

11. Zelle nach Anspruch 10, **dadurch gekennzeichnet, dass** sie in ihrem Chloroplastengenom Sequenzen von mindestens 200 aufeinanderfolgenden Basenpaaren umfasst, die von *Raphanus sativus* stammen.

## Claims

1. Plant of the species *Diplotaxis tenuifolia,* wherein said plant carries cytoplasmic male sterility, wherein its mitochondrial genome comprises the DNA sequence corresponding to nucleotides 1673 to 2089 of SEQ ID N°1, or a sequence having at least 70% identity with said DNA sequence, and wherein it has no symptoms of chlorosis under normal culture conditions, in particular no discoloration of leaves due to a lack of chlorophyll.

**2.** Plant according to claim 1, wherein it is a hybrid plant.

**3.** Plant according to claim 1, wherein, in its cytoplasmic genome, it comprises a sequence of at least 200 consecutive base pairs originating from the chloroplastic genome of *Raphanus sativus.*

**4.** Plant according to claim 3, wherein an amplification fragment with the following size is obtained when the corresponding primers are used to amplify its chloroplastic genome by PCR:

- 193 base pairs when the primers SEQ ID N°4 and 5 are used; and/or
- 264 base pairs when the primers SEQ ID N°16 and 17 are used; and/or
- 180 base pairs when the primers SEQ ID N°14 and 15 are used; and/or
- 319 base pairs when the primers SEQ ID N°18 and 19 are used; and/or
- 303 base pairs when the primers SEQ ID N°6 and 7 are used; and/or
- 183 base pairs when the primers SEQ ID N°12 and 13 are used.

**5.** Plant according to claim 1, wherein an amplification fragment with the following size is obtained when the corresponding primers are used to amplify its mitochondrial genome by PCR:

- 512 base pairs when the primers SEQ ID N°22 and 23 are used and /or
- 401 base pairs when the primers SEQ ID N°24 and 25 are used.

**6.** Plant according to one of claims 1 to 5, wherein its cells have the genomic DNA of *Diplotaxis tenuifolia* and the mitochondrial and chloroplastic DNA of *Raphanus sativus.*

**7.** Seed of the species *Diplotaxis tenuifolia,* wherein said seed carries cytoplasmic male sterility, and wherein its mitochondrial genome comprises the DNA sequence corresponding to nucleotides 1673 to 2089 of SEQ ID N°1, or a sequence having at least 70% identity with said DNA sequence.

**8.** Seed according to claim 8, wherein it comprises in its chloroplastic genome sequences of at least 200 consecutive base pairs originating from *Raphanus sativus.*

**9.** Cell of a plant of the species *Diplotaxis tenuifolia,* wherein it carries cytoplasmic male sterility, and wherein its mitochondrial genome comprises the DNA sequence corresponding to nucleotides 1673 to 2089 of SEQ ID N°1, or a sequence having at least 70% identity with said DNA sequence.

**10.** Cell according to claim 9, originating from a plant in accordance with any one of claims 1 to 6 or a seed in accordance with any one of claims 7 to 8.

**11.** Cell according to claim 10, wherein it comprises in its chloroplastic genome sequences of at least 200 consecutive base pairs originating from *Raphanus sativus.*

```
   1 aagcttgaag ggtaatatct tcgccaccct cccgactcgc aatccccaat gtggctttcc
  61 tcacttcggt aagttacaag gatgaatgca aatagcaagg cgctatgctg cgtgaagtga
 121 gaccggctgt cctaagttaa gttgcttcct tattcattaa tgatcttgct cagtaggagg
 181 gcttttcccc ttctgtgcag cctgatttta tctctggaaa tgagggtgcc ctcgattgca
 241 atttttcatc gaatatgatg gattcccccc atggtgagaa ggttgagtta cagactccgt
 301 tggctttcgc aagacagtct cgaaagttcc gaaatctatg gttgatgtct cactcgaact
 361 ctatccctga ctttcttcgt ccgagcggcc cctctaggag ccgatgcggg aaggaagaag
 421 gatttatttc taccattaaa taattcgcaa tagttaacca agcagtagcc aagaacaaag
 481 aagcaagcca agatcaatgg gggggtcgc cccagtcaaa aatggtaaga aggagtagca
 541 tcaataacat tcattgtatg tacgcctccc agcgatccac aggcatctgt agcatgttgt
 601 acccgagagt tattttggct gtgtctgtta caccatggac aataatctta gtcggagtca
 661 aattccttac ctttccaccc aaagctgaac atatccgcac agatattcca ttttttttat
 721 tgaggatcca ttttcgaact gaactactca tgcttaggca aaacaagcaa gggagttgtt
 781 aataaggagc tagctacagt gctcctgcgg gagggttccc gtgcttatta aggagccggg
 841 cagctacgca acacttcctt gcaactcata cctactaaca aactgtttac tctttttaag
 901 gagttagctg catttccctg cggaggtacg tacgcaatca aagcagcagg gcagcttcgc
 961 aacacctgct tcaacttcat gcacattagc aacaagattg ggtagttgat tgttgggagg
1021 atagctgcag ctccctacgg gagtgaacta cagttccagg gggagcacag caagggccaa
1081 taccggctgt gaggcgcgta gcgggaagag atgtatggta agggatagct gtttaaccat
1141 ttgtaatgga atgggatgtt gatcctcctt ggaataatac gtatataaga agattttcat
```

1201 tccagttgga aagcaatcga gaaaacgccg cccaaatacg gttcgcc**acg tgtagccctg**
1261 **tatggact**cg cgaagcaggt ctccggtcgg tgtccaagat ttgatctaac tattgagtga

```
1321 ggactactta ccgattgata gaataatacg tatataagaa gaaggctgct ttgtggagtg
1381 atctttctcg aaatgaatta agtaagggcg ctatgttcag attctgaacc aaagcactag
1441 ttgaggtctg aaagccttat gagcagaagt aataaatacc tcggggaaga agcggggtag
1501 aggaattggt caactcatca ggctcatgac ctgaagatta caggttcaaa tcctgtcccc
```

1561 gcaccgtagt ttcattct**gc atcactctcc ctgtcgttat cg**acctcgca aggtttttga
1621 aacggcc**gaa acgggaagtg acaatacc**gc ttttcttcag catataaatg caatgattac

```
1681 ctttttcgaa aaattgtcca ctttttgtca taatctcact cctactgaat gtaaagttag
1741 tgtaataagt ttctttcttt tagcttttt actaatggcc catatttggc taagctggtt
1801 ttctaacaac caacattgtt tacgaaccat gagacatcta gagaagttaa aaattccata
1861 tgaatttcag tatgggtggc taggtgtcaa aattacaata aaatcaaatg tacctaacga
1921 tgaagtgacg aaaaaagtct cacctatcat taaaggggaa atagagggga aagaggaaaa
1981 aaaagagggg aaaggggaaa tagaggggaa agaggaaaaa aaagagggga aaggggaaat
```

2041 agaggggaaa gaggaaaaaa aagagg**tgga aaatggaccg agaaaataat** gctttgtgaa

```
2101 cccaattgct ttgacaaaaa taaagaaaga agcaaaatct cattcaattt gaaatagaag
2161 agatctctat gccccctgtt cttggttttc tcccatgctt ttgttggtca acaaccaacc
2221 acaactttct atagttcttc actactccta gaggctttgg taatgaagct gtctggaggg
2281 atttgttgaa atcaattaat ctaatcatgc ctcaactgga taaattcact tatttttcac
2341 aattcttctg gttatgcctt ttcttcttta ctttctatat tttcatatgc aatgatggag
2401 atggagtact tgggatcagc agaattctaa aactacggaa ccaactgctt tcacaccggg
2461 ggaagaccat ccagagcaag gaccccaaca gtttggaaga tctcttgaga aaaggtttta
2521 gcactggtgt atcctatatg tatgctagtt tattcgaagt atcccaatgg tgtaaggccg
2581 tcgacttatt gggaaaaagg aggaaaatca ctttgatctc ttgtttcgga gaaataagtg
2641 gctcacgagg aatggaaaga aacatattat ataatctatc gaagtcctct ccttcaaata
2701 ctggaaggtg gatcacttgt aggaattgta ggaatgacat aatgctaatc catgctgtac
2761 atggccaagg aagcataaaa tgattctttc attctataga tacctctggt aggtaaagca
2821 ctctactgtg ctttattgaa agttcccatc gcgggggcag ggatacttgc cttcgcggtt
2881 cgactttctt ttcaggcttg actcattatt ttccggtcct ctcacacccc tttagagctc
2941 tttatgatgc ccactgagta agattcgggg gcttaccggc gcagaagctc attctgaacc
3001 gcgggaacct tcgtctcttc gacacaaacg ttttatgaag aggctgatgg tgatgaggat
3061 ccatgcgcac tcaaataaaa gtagcttgcg tattgggtta tccacggtgt taggtgctcc
3121 attgcacctc atgtggaggt aggataattt ggactctttt ggagcactat actccgaaag
3181 atcctatcct tcctcctctt ctttcagtcg agacttcctc ctagtgaggt gttgcctatc
3241 caggtatgga agtattcaat gaatacactc tgtaccatgg gtggatgaag ctt
```

Z18896 SEQ ID N°1

**FIGURE 1**

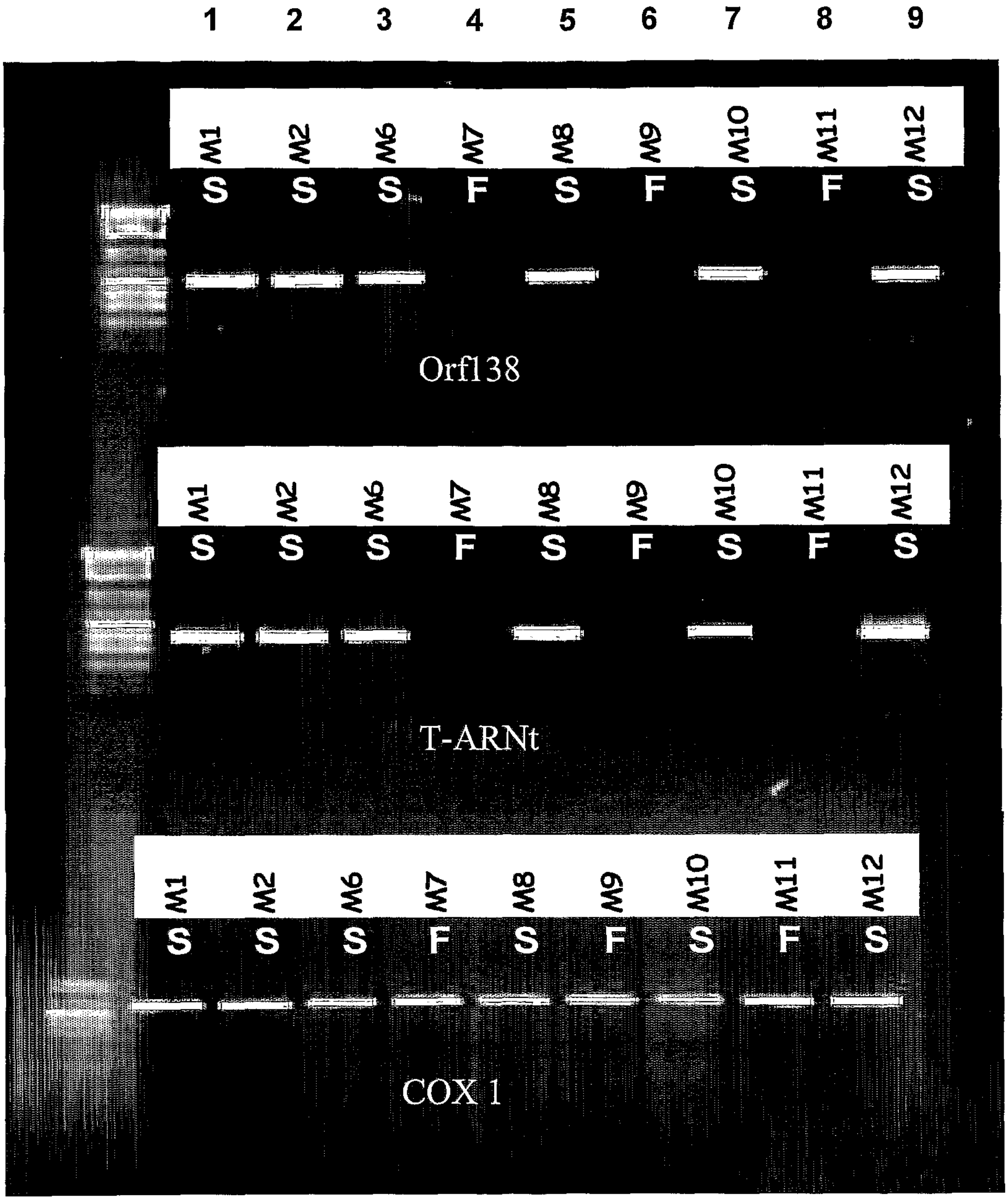
1
2
3
4
5
6
7
8
9
M1 M2 M6 M7 M8 M9 M10 M11 M12
S S S F S F S F S
Orf138
M1 M2 M6 M7 M8 M9 M10 M11 M12
S S S F S F S F S
T-ARNt
M1 M2 M6 M7 M8 M9 M10 M11 M12
S S S F S F S F S
COX 1

**FIGURE 2**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 549726 A
- EP 0549726 A


### Littérature non-brevet citée dans la description


- **CAO J. ; EARLE, E.D.** *Plant Cell Report,* 2003, vol. 21, 789-796
- **CHU C.C.** The N- medium and its application to anther culture of Cereal crops. *Proc. Symp. Plant Tissue culture, Peking,* 1978, vol. 43
- **CHU C.C et al.** Establishement of an efficient medium for anther culture of Rice through comparative experiments on the nitrogen sources,. *Scienta Sinic,* 1975, vol. 18, 659
- **FLANNERY M.L. et al.** Plastid genome characterisation in Brassica and Brassicaceae using a new set of nine SSRs. *Theor Appl Genet,* 2006, vol. 113, 1221-1231
- **MATSUZAWA et al.** Male sterility in alloplasmic Brassica rapa L. carrying Eruca sativa cytoplasm. *Plant Breeding,* 1999, vol. 118, 82-84
- **MURASHIGE, T. ; SKOOG F.** A revised medium for rapid growth and bioassays with tobacco tissue cultures. *Physiol. Plant,* 1962, vol. 15, 473
- **RAHMAN, M.H.** Optimum age of siliques for rescue of hybrid embryos from crosses between Brassica oleracea, B. rapa and B. carinata. *Canadian Journal of Plant Science,* 2004, vol. 84 (4), 965-969
- **WOO BANG, S. et al.** Production of Intergeneric Hybrids between the C3-C4 Intermediate Species Diplotaxis tenuifolia (L.) DC. and Raphanus sativus L. *Breeding Sciences,* 2003, vol. 53, 231-236